# EUROPEAN PATENT SPECIFICATION

(11) **EP 2 822 532 B1**
(45) Date of publication and mention of the grant of the patent: **19.09.2018**
(21) Application number: 13712499.6
(22) Date of filing: 06.03.2013
(51) Int. Cl.: A61K 8/368, A61Q 19/00, A61K 8/87, A61K 8/04, A61P 17/10, C08K 5/09, C08L 75/04

(54) **AQUEOUS POLYURETHANE DISPERSION IN THE TREATMENT OF ACNE**
WÄSSRIGE POLYURETHAN-DISPERSION FÜR DIE BEHANDLUNG VON AKNE
UN DISPÉRSION AQUEUSE DE POLYURÉTHANE POUR LE TRAITEMENT DE L'ACNÉ

(30) Priority: 09.03.2012 US 201261608814 P
(43) Date of publication of application: 14.01.2015
(73) Proprietor: Covestro Deutschland AG, 51373 Leverkusen (DE)
(72) Inventor: VIALA, Sophie, 50935 Köln (DE); DÖRR, Sebastian, 40593 Düsseldorf (DE); BEREZKIN, Yuliya, Pittsburgh, Pennsylvania 15241 (US)
(74) Representative: Levpat
(86) International application number: PCT/EP2013/054527
(87) International publication number: WO 2013/131969

(56) References cited:
- EP-A1- 2 380 557
- EP-A1- 2 395 036
- WO-A2-2007/031883
- WO-A2-2009/135857

## Description

The present invention describes a surprising effect of an aqueous polyurethane dispersion on the efficacy of active ingredients in the anti-acne treatment. The aqueous polyurethane dispersion was able to reduce irritation and substantially enhance efficacy of the salicylic acid and/or salicylic acid derivative in the topical anti-acne serum.

The use of the variety of active ingredients in cosmetic and OTC (Over-the -Counter) products had become a necessity in order to achieve a wide range of cosmetic and therapeutical effects to satisfy as ever more educated and demanding consumer base. Most of these active ingredients, such as vitamins, AHAs, enzymes and plant extracts are known for their sensitivity to anti-oxidants, light and water. Many actives used in OTC products, such as salicylic acid, benzoyl peroxide, retinoids, at the efficacious levels are harsh for sensitive and mature skin, causing irritation, burning sensation and excessive dryness. Formulating with those actives presents another challenge of maintaining efficacy of these actives over a prolonged period ensuring its long-lasting effect in the topical application.

Various encapsulating techniques and delivery systems are proposed to mediate either preservation of actives or their efficacy enhancement.

Specifically, the use of polymers to control delivery of actives and/or to modify treated surfaces for enhancing efficacy of actives are described in the prior art.

P. Thau in "Controlled delivery and enhancement of Topical Activity of salicylic Acid", in Delivery Systems Handbook for Personal care and Cosmetic Products, MR Rosen, ed. William Andrew, Inc,p.873-880, reviews contemporary technologies and vehicles to modify delivery and activity of salicylic acid. Among delivery technologies categories, one of the polymeric complexation methods is described as using a low molecular weight PEG-8/SMDI copolymers, number-average molecular weight 1,800 g/mol (polyurethane oligomers) that associate with stratum corneum, thus increasing deposition of salicylic acid in the upper layers of stratum corneum, which provides potential for increased keratolytic activity. Further, this technology suggests a decrease of the permeation of salicylic acid through pigskin via binding of salicylic acid to the oligomer which is a critical for altering of salicylic acid permeation kinetics.

Most encapsulating or polymer entrapment technologies involve a release mechanism through a combination of friction upon application to skin and controlled diffusion of salicylic acid from the various types of polymeric shells. These types of technology are often offered in a form of free powder.

US 8,039,020 B2 teaches encapsulating technique that emphasizes the core-shell structure of the capsule containing active ingredient. The invention focuses on preserving unstable active ingredients, such as benzoyl peroxide, and on stabilizing cosmetic formulation.

US 7,166,235 describes the use of siloxane polymers with a pendant carboxyl groups for hydrophobically modifying cationic surfaces and possibly acting as carriers to deposit actives improving their retention and efficacy.

US 8,017,150 describes a PO-based polymers and their blend with cellulosic polymers that form a uniformed film which facilitates an even distribution of actives and prevents their self-agglomeration. The patent teaches an importance of film's uniformity and even distribution of drugs or actives in it for achieving maximum drug efficacy. The proposed films are water soluble and hydrophilic. The films are used in a dry form to deliver actives or drugs as an alternative to tablets.

EP 0 998 914 A1 describes compositions for enhancing and regulating the penetration of topical skin agents into the epidermal and dermal layers of the skin comprise at least one active ingredient which is hydrophilic or hydrophobic, a polymeric emulsifier and, alternatively, a sugar or a polyoxyethylene alcohol. The compositions are mild and non-irritating despite the increased penetration of topical active agents.

WO 1999/047 072 A1 describes new hydrophilic polyether polyurethane polymers containing carboxylic acid in diol component, having good adhesion, slip and strength. Claims an article of manufacture comprising a substrate carrying a layer of (I) and an active agent (A) selected from pharmacologically active agents. The articles are medical devices, adsorbents, gloves and body implants.

US 6,177,068 B1 teaches about production of cross-linked vinylamide polymer emulsion, for topical cosmetic applications, involves mixing vinylamide polymer in oil solvent, free radical initiator, water and cosmetic agent under specific condition

US 2011/0076243 A1 discloses are hydrophilic polymers such as polysaccharides, including hyaluronic acid, modified by reaction with epoxy-functional-silicones. Hydrophobic silicon, which contains chemically active groups covalently attach to the backbone of the hydrophilic polymer and gives these new, modified polymers the ability to dissolve hydrophobic compounds including oils, drugs, and vitamins, while maintaining the hydrophilic properties and benefits of the unmodified polymer. With respect to topical applications these polymers substantially increase the stability of formulations and provide for ease of preparation. The products can be used for various applications including cosmetic, medical, and drug delivery applications.

US 2010/0008884 A1 describes the process of producing a hydrophobically modified polymer that is prepared by incorporating hydrophobic moieties into a polymer composed acrylamide, cationic monomers and optionally anionic monomers. The composition including hydrophobically modified polyacrylamide that enhances properties such as creaminess, slip, feel, viscosity and moisturization depending on the type of hydrophobic moiety incorporated into the polymer.

The use of salicylic acid in the treatment of common or teen acne is known. For example, U.S. Pat. No. 4,665,063 describes the use of topically applied aspirin (acetyl salicylic acid) for treating common acne; and U.S. Pat. No. 4,891,227 describes the use of pads for applying anti-acne products containing salicylic acid for oily skin. These patents describe state-of-art compositions which emphasize aggressive chemical and physical treatment suitable for teen acne, without addressing the suitability for adult acne and/or the need for mildness.

U.S. 4,800,197 describes a combination of salicylic acid and an anionic taurate surfactant, specifically sodium methyl cocoyl taurate or sodium methyl oleoyl taurate. U.S. 5,296,476 describes the specific use of salicylic acid in combination with calcium citrate. Again, these treatment modalities are designed for aggressive, physical cleansing, which assumes that the individual indicators are normal, young and oily skin.

U.S. 5,569,651 teaches the use of a salicylic acid cream and lotions whose pH is adjusted to from about 3.8 to 4.5 using ammonium hydroxide. U.S. 5,871,764 discloses a salicylic acid powder formulation having a pH of from about 3 to about 4. U.S. 5,612,324 discloses salicylic acid solutions, gels and pads having a pH of from about 2 to about 6.5.

Additional examples of salicylic acid compositions wherein pH of the composition was controlled may be found in the following U.S. 5,756,119 (pH of less than 5); U.S. 5,549,888 (pH of 2-5.4); U.S. 4,294,852 (pH of 2-6.5); U.S. 5,702,688 (pH less than or equal to 4.2); U.S. 4,800,197 (pH of 2-3.5); and U.S. 5,958,436 (pH of 1-6).

While many technologies claim to improve deposition and retention of salicylic acid on the top layers of stratum corneum, controlling its release and enhancing efficacy, most of these technologies are beneficial only in various degrees of performance, safety, aesthetics and value to the consumer and to the cosmetic formulator. A great majority of the proposed solutions show advantages in one or in a subset of the above mentioned properties.

A need for a multifunctional, safe, effective and easy to use delivery system still exists to formulate mild and efficacious cosmetic and OTC products for sophisticated consumers.

The present invention describes an aqueous polyurethane film former and the compositions containing such film former for improving efficacy and reducing irritation of OTC products. A polyurethane polymer used at preferably 0.1 to 15 % by weight, more preferably 0.5 to 10% by weight, even more preferably 1.0 to 5.0 % by weight, in each case based on the total weight of the composition in the anti-acne composition reduced redness and size of the acne lesion, also improved anti-flammatory activity of active ingredients in the formulation. The additional benefits of the film former include its pleasantly natural feel on skin, comfortable wear and non-comedogenic properties. It was demonstrated that the film former alone was capable to boost efficacy of the anti-flammatory ingredients in the anti-acne serum. The polymer poses a unique combination of properties that allows for achieving significant difference in performance from the standard anti-acne composition. The polymer is an inert, water dispersible high molecular weight polyurethane that forms exceptionally flexible and clear film.

Thus, the presently claimed invention is in one aspect related to an aqueous composition comprising at least one aqueous polyurethane dispersion and salicylic acid and/or at least one salicylic acid derivative as set out in claim 1.

The salicylic acid derivative is selected from the group consisting of acetaminosalol, aspirin, balsalazide, benorylate, calcium acetylsalicylate, diflunisal, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salicylamide O-acetic acid, salicylsulfuric acid, salsalate, sodium salicylate and sulfasalazine.

As used herein the term "acne" refers to any and all forms of acne as well as acneform conditions such as, without limitation, folliculitis keratosis pilaris. Acne is a broad clinical syndrome most frequently occurring at puberty in both men and women. It may last through life, and consists of lesions most typically on the face and trunk, and consists of papules, pustules, comedones (open and closed), cysts, and microcysts.

Used in the composition of the present invention is an aqueous dispersion where the polyurethane is obtainable by reacting one or more water-insoluble, non-water-dispersible isocyanate-functional polyurethane prepolymers A) which have neither ionic nor ionogenic groups, with at least one or more amino-functional compounds B), which include at least one amino-functional compound B2) which has ionic and/or ionogenic groups.

Within the context of the invention, the term "water-insoluble, non-water-dispersible polyurethane prepolymer" means in particular that the solubility in water of the prepolymer used according to the invention at 23°C is less than 10 g/litre, more preferably less than 5 g/litre, and the prepolymer does not produce a sedimentation-stable dispersion in water, in particular deionized water, at 23°. In other words, the prepolymer settles out upon attempting to disperse it in water.

Preferably, the polyurethane prepolymer A) used according to the invention has terminal isocyanate groups, i.e. the isocyanate groups are at the chain ends of the prepolymer. All of the chain ends of a polymer particularly preferably have isocyanate groups.

Furthermore, the polyurethane prepolymer A) used according to the invention preferably has neither ionic nor ionogenic (capable of forming ionic groups) groups, i.e. the content of ionic and ionogenic groups is expediently below 15 milliequivalents per 100 g of polyurethane prepolymer A), preferably below 5 milliequivalents, particularly preferably below 1 milliequivalent and very particularly preferably below 0.1 milliequivalent per 100 g of polyurethane prepolymer A).

The amino-functional compounds B) include at least one amino-functional compound B2) which has ionic and/or ionogenic (ion-forming) groups. The ionic and/or ionogenic group used is particularly preferably the sulphonate or the sulphonic acid group, yet more preferably the sodium sulphonate group.

In a further preferred embodiment of the invention, the amino-functional compounds B) include both amino-functional compounds B2) which have ionic and/or ionogenic group, and also amino-functional compounds B1) which have no ionic or ionogenic group.

Accordingly, polyurethanes within the context of the invention are polymeric compounds which have at least two, preferably at least three, repeat units containing urethane groups:

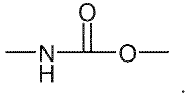

According to the invention, also included are those polyurethanes which, as a result of the preparation, also have repeat units containing urea groups: 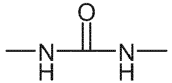 as are formed in particular in the reaction of the isocyanate-terminated prepolymers A) with the amino-functional compounds B).

The skin care products according to the invention are preferably water-containing, i.e. aqueous, compositions in which the polyurethane is present in dispersed form, i.e. not in dissolved form. In general, besides any other liquid media which may be present, such as, for example, solvents, water forms the main constituent (> 50% by weight) of the dispersion media, based on the total amount of the liquid dispersion media in the cosmetic compositions according to the invention, and in some cases also forms the sole liquid dispersion medium.

The skin care products according to the invention preferably have a content of volatile organic compounds (VOCs) of less than 80% by weight, more preferably of less than 55% by weight, even more preferably of less than 40% by weight, based on the skin care product.

The aqueous polyurethane dispersions used for the preparation of the skin care products according to the invention preferably have a content of volatile organic compounds (VOCs) of less than 10% by weight, more preferably of less than 3% by weight, even more preferably of less than 1% by weight, based on the aqueous polyurethane dispersion.

The content of volatile organic compounds (VOCs) is determined within the context of the present invention in particular by gas chromatographic analysis.

The non-water-soluble and non-water-dispersible, isocyanate-functional polyurethane prepolymers used according to the invention have neither ionic nor ionogenic groups. The insolubility in water and/or lack of dispersibility in water refers to deionized water without the addition of surfactants. Within the context of the present invention this means that the proportion of ionic and/or ionogenic (ion-forming) groups, such as, in particular, anionic groups, such as carboxylate or sulphonate, or of cationic groups is less than 15 milliequivalents per 100 g of polyurethane prepolymer A), preferably less than 5 milliequivalents, particularly preferably less than 1 milliequivalent and very particularly preferably less than 0.1 milliequivalent per 100 g of polyurethane prepolymer A).

In the case of acidic ionic and/or ionogenic groups, the acid number of the prepolymer is expediently below 30 mg of KOH/g of prepolymer, preferably below 10 mg of KOH/g of prepolymer. The acid number indicates the mass of potassium hydroxide in mg which is required to neutralize 1 g of the sample under investigation (measurement in accordance with DIN EN ISO 211). The neutralized acids, i.e. the corresponding salts, naturally have no acid number or a reduced acid number. According to the invention, the acid number of the corresponding free acid is decisive here.

The prepolymers A) used for the preparation of the polyurethanes are preferably obtainable by reacting one or more polyols selected from the group which consists of polyether polyols, polycarbonate polyols, polyether polycarbonate polyols and/or polyester polyols, and polyisocyanates, as is explained in more detail below.

The polyurethanes present in the skin care products according to the invention accordingly comprise, via the prepolymer A), preferably at least one sequence selected from the group which consists of: polyether, polycarbonate, polyether-polycarbonate and polyester sequences. According to the invention, this means in particular that the polyurethanes contain repeat units containing ether groups and/or carbonate groups or ester groups. The polyurethanes can contain, for example, exclusively polyether sequences or exclusively polycarbonate sequences or exclusively polyester sequences. However, they can also have both polyether and polycarbonate sequences, as are formed, for example, during the preparation of polycarbonate polyols using polyetherdiols, as is described in more detail below. In addition, they can have polyether-polycarbonate sequences which arise from the use of polyether-polycarbonate polyols, as described in more detail below.

Particularly preferred polyurethanes are obtained using polymeric polyether polyols and/or polymeric polycarbonate polyols and/or polyether-polycarbonate polyols or polyester polyols, each of which have number-average molecular weights of preferably about 400 to about 6000 g/mol (here and in the case of the molecular weight data below, determined by gel permeation chromatography relative to polystyrene standard in tetrahydrofuran at 23°C). Their use during the preparation of the polyurethanes or polyurethane prepolymers leads, as a result of the reaction with polyisocyanates, to the formation of corresponding polyether and/or polycarbonate and/or polyether-polycarbonate sequences or polyester sequences in the polyurethanes with a corresponding molecular weight of these sequences. According to the invention, particular preference is given to polyurethanes which are obtained from polymeric polyetherdiols and/or polymeric polycarbonatediols and/or polyether-polycarbonate polyols or polyester polyols with a linear structure.

The polyurethanes according to the invention are preferably linear molecules, but may also be branched, which is less preferred.

The number-average molecular weight of the polyurethanes preferably used according to the invention is, for example, about 1000 to 200 000, preferably from 5000 to 150 000.

The polyurethanes present in the skin care products according to the invention are added to the specified compositions in particular in the form of aqueous dispersions.

Preferred polyurethanes or polyurethane dispersions to be used according to the invention are obtainable by
A) preparing isocyanate-functional prepolymers of
   A1) organic polyisocyanates,
   A2) polymeric polyols, preferably with number-average molecular weights of from 400 to 8000 g/mol (here and for the molecular weight data below, determined by gel permeation chromatography relative to polystyrene standard in tetrahydrofuran at 23°C), more preferably 400 to 6000 g/mol and particularly preferably from 600 to 3000 g/mol, and OH functionalities of preferably 1.5 to 6, more preferably 1.8 to 3, particularly preferably from 1.9 to 2.1,
   A3) optionally hydroxy-functional compounds with molecular weights of preferably 62 to 399 g/mol, and
   A4) optionally nonionic hydrophilizing agents,
   and
B) then reacting some or all of their free NCO groups with one or more amino-functional compounds B2) which has ionic and/or ionogenic groups.

The polyurethanes used according to the invention are preferably dispersed in water before, during or after step B).

The reaction with a diamine or two or more diamines in step B) particularly preferably takes place with chain extension. In this connection, monofunctional amines can additionally be added as chain terminators to control the molecular weight.

As component B), in particular amines can be used which have no ionic or ionogenic, such as anionically hydrophilizing groups (component B1 below)). Amines which have ionic or ionogenic, such as, in particular, anionically hydrophilizing groups (component B2 below)) are always present,.

Preferably, in step B) of the reaction of the prepolymer, a mixture of component B1) and component B2) is reacted. By using component B1) it is possible to build up a high molar mass without the viscosity of the previously prepared isocyanate-functional prepolymer increasing to a degree which would be an obstacle to processing. By using the combination of components B1) and B2) it is possible to achieve an optimum balance between hydrophilicity and chain length and thus establish a pleasant skin feel.

The polyurethanes used according to the invention preferably have anionic groups, preferably sulphonate groups. These anionic groups are introduced into the polyurethanes used according to the invention via the amine component B2) reacted in step B). The polyurethanes used according to the invention optionally additionally have nonionic components for hydrophilization. Exclusively sulphonate groups are particularly preferably present in the polyurethanes used according to the invention for the hydrophilization; these are introduced into the polyurethane via corresponding diamines as component B2).

In order to achieve a good sedimentation stability, the number-average particle size of the special polyurethane dispersions is preferably less than 750 nm, particularly preferably less than 500 nm, determined by means of laser correlation spectroscopy following dilution with deionized water (instrument: Malvern Zetasizer 1000, Malvern Inst. Limited).

The solids content of the polyurethane dispersions which is preferably used for preparing the skin care product of the invention is generally 10 to 70% by weight, preferably 30 to 65% by weight, particularly preferably 40 to 60% by weight. The solids contents are ascertained by heating a weighed sample at 125°C to constant weight. At constant weight, the solid-body content is calculated by reweighing the sample.

Preferably, these polyurethane dispersions have less than 5% by weight, particularly preferably less than 0.2% by weight, based on the mass of the dispersions, of unbonded organic amines. The content in the skin care products is correspondingly yet lower.

Suitable polyisocyanates of component A1) are in particular the aliphatic, aromatic or cycloaliphatic polyisocyanates with an NCO functionality of greater than or equal to 2 known per se to the person skilled in the art.

Examples of such suitable polyisocyanates are 1,4-butylene diisocyanate, 1,6-hexamethylene diisocyanate (HDI), isophorone diisocyanate (IPDI), 2,2,4- and/or 2,4,4-trimethylhexamethylene diisocyanate, the isomeric bis(4,4'-isocyanatocyclohexyl)methanes or mixtures thereof of any desired isomer content, 1,4-cyclohexylene diisocyanate, 4-isocyanatomethyl-1,8-octane diisocyanate (nonane triisocyanate), 1,4-phenylene diisocyanate, 2,4- and/or 2,6-tolylene diisocyanate, 1,5-naphthylene diisocyanate, 2,2'- and/or 2,4'- and/or 4,4'-diphenylmethane diisocyanate, 1,3- and/or 1,4-bis(2-isocyanatoprop-2-yl)benzene (TMXDI), 1,3-bis(isocyanatomethyl)benzene (XDI), and alkyl 2,6-diisocyanatohexanoates (lysine diisocyanates) with C1-C8-alkyl groups.

Besides the aforementioned polyisocyanates, it is also possible to use modified diisocyanates which have a functionality of ≥ 2 with uretdione, isocyanurate, urethane, allophanate, biuret, iminooxadiazinedione or oxadiazinetrione structure, and also mixtures of these proportionately.

They are preferably polyisocyanates or polyisocyanate mixtures of the type specified above with exclusively aliphatically or cycloaliphatically bonded isocyanate groups or mixtures of these and an average NCO functionality of the mixture of from 2 to 4, preferably 2 to 2.6 and particularly preferably 2 to 2.4, very particularly preferably 2.

Hexamethylene diisocyanate, isophorone diisocyanate or the isomeric bis(4,4'-isocyanatocyclohexyl)methanes, and mixtures of the aforementioned diisocyanates are particularly preferably used in A1).

In A2), polymeric polyols with a number-average molecular weight Mₙ of preferably 400 to 8000 g/mol, more preferably from 400 to 6000 g/mol and particularly preferably from 600 to 3000 g/mol are used. These preferably have an OH functionality of from 1.5 to 6, particularly preferably from 1.8 to 3, very particularly preferably from 1.9 to 2.1.

The expression "polymeric" polyols means here in particular that the specified polyols have at least two, more preferably at least three, repeat units joined together.

Such polymeric polyols are the polyester polyols, polyacrylate polyols, polyurethane polyols, polycarbonate polyols, polyether polyols, polyester polyacrylate polyols, polyurethane polyacrylate polyols, polyurethane polyester polyols, polyurethane polyether polyols, polyurethane polycarbonate polyols and polyester polycarbonate polyols known per se in polyurethane coating technology. These can be used in A2) individually or in any desired mixtures with one another.

The preferably used polyester polyols are the polycondensates known per se of di- and optionally tri- and tetraols and di- and optionally tri- and tetracarboxylic acids or hydroxycarboxylic acids or lactones. Instead of the free polycarboxylic acids, it is also possible to use the corresponding polycarboxylic acid anhydrides or corresponding polycarboxylic acid esters of lower alcohols for the preparation of the polyesters.

Examples of suitable diols are ethylene glycol, butylene glycol, diethylene glycol, triethylene glycol, polyalkylene glycols, such as polyethylene glycol, also 1,2-propanediol, 1,3-propanediol, butanediol(1,3), butanediol(1,4), hexanediol(1,6) and isomers, neopentyl glycol or hydroxypivalic neopentyl glycol ester, where hexanediol(1,6) and isomers, butanediol(1,4), neopentyl glycol and hydroxypivalic neopentyl glycol ester are preferred. In addition, polyols such as trimethylolpropane, glycerol, erythritol, pentaerythritol, trimethylolbenzene or trishydroxyethyl isocyanurate can also be used.

Dicarboxylic acids which can be used are phthalic acid, isophthalic acid, terephthalic acid, tetrahydrophthalic acid, hexahydrophthalic acid, cyclohexanedicarboxylic acid, adipic acid, azelaic acid, sebacic acid, glutaric acid, tetrachlorophthalic acid, maleic acid, fumaric acid, itaconic acid, malonic acid, suberic acid, 2-methylsuccinic acid, 3,3-diethylglutaric acid and/or 2,2-dimethylsuccinic acid. The corresponding anhydrides may also be used as acid source.

If the average functionality of the polyol to be esterified is > than 2, monocarboxylic acids, such as benzoic acid and hexanecarboxylic acid, can additionally also be co-used.

Preferred acids are aliphatic or aromatic acids of the type specified above. Particular preference is given to adipic acid, isophthalic acid and phthalic acid.

Hydroxycarboxylic acids which can be co-used as reactants in the preparation of a polyester polyol with terminal hydroxyl groups are, for example, hydroxycaproic acid, hydroxybutyric acid, hydroxydecanoic acid, hydroxystearic acid and the like. Suitable lactones are caprolactone, butyrolactone and homologues. Preference is given to caprolactone.

According to the invention, particularly preferred components A2) for the preparation of the polyurethanes are polyester polyols with a number-average molecular weight of from 600 to 3000 g/mol, in particular aliphatic polyester polyols based on aliphatic carboxylic acids and aliphatic polyols, in particular based on adipic acid and aliphatic alcohols, such as hexanediol and/or neopentyl glycol.

Polycarbonates having hydroxyl groups, preferably polycarbonatediols, with number-average molecular weights Mₙ of from preferably 400 to 8000 g/mol, preferably 600 to 3000 g/mol can likewise be used as component A2). These are obtainable by reacting carbonic acid derivatives, such as diphenyl carbonate, dimethyl carbonate or phosgene, with polyols, preferably diols.

Examples of such diols are ethylene glycol, 1,2- and 1,3-propanediol, 1,3- and 1,4-butanediol, 1,6-hexanediol, 1,8-octanediol, neopentyl glycol, 1,4-bishydroxymethylcyclohexane, 2-methyl-1,3-propanediol, 2,2,4-trimethylpentanediol-1,3, dipropylene glycol, polypropylene glycols, dibutylene glycol, polybutylene glycols, bisphenol A and lactone-modified diols of the type specified above.

Preferably, the diol component comprises 40 to 100% by weight of hexanediol, preference being given to 1,6-hexanediol and/or hexanediol derivatives. Such hexanediol derivatives are based on hexanediol and, besides terminal OH groups, have ester or ether groups. Such derivatives are obtainable by reacting hexanediol with excess caprolactone or by etherifying hexanediol with itself to give the di- or trihexylene glycol.

Instead of or in addition to the pure polycarbonatediols, it is also possible to use polyether- polycarbonatediols in A2).

Polycarbonates having hydroxyl groups preferably have a linear structure.

Polyether polyols can likewise be used as component A2).

For example, the polytetramethylene glycol polyethers known per se in polyurethane chemistry, as are obtainable through polymerization of tetrahydrofuran by means of cationic ring opening, are particularly suitable.

Likewise suitable polyether polyols are the addition products, known per se, of styrene oxide, ethylene oxide, propylene oxide, butylene oxide and/or epichlorohydrin onto di- or polyfunctional starter molecules. Thus, in particular polyalkylene glycols, such as polyethylene glycols, polypropylene glycols and/or polybutylene glycols, can be used, in particular those with the preferred molecular weights specified above.

Suitable starter molecules which can be used are all compounds known according to the prior art, such as, for example, water, butyl diglycol, glycerol, diethylene glycol, trimethyolpropane, propylene glycol, sorbitol, ethylenediamine, triethanolamine 1,4-butanediol.

Particularly preferred components in A2) are polytetramethylene glycol polyethers and polycarbonate polyols and mixtures thereof and particularly preferably polytetramethylene glycol polyethers.

In preferred embodiments of the invention, component A2) is accordingly:
- mixtures comprising at least one polyether polyol and at least one polycarbonate polyol,
- mixtures comprising more than one polyether polyol, or a mixture of two or more polyether polyols with different molecular weights, which are in particular polytetramethylene glycol) polyether polyols (such as HO-(CH₂-CH₂-CH₂-CH₂-O)ₓ-H),

- mixtures comprising more than one polyether polyol and at least one polycarbonate polyol, and also
- particularly preferably polyester polyols with a number-average molecular weight of from 600 to 3000 g/mol, in particular aliphatic polyester polyols based on aliphatic carboxylic acids and aliphatic polyols, in particular based on adipic acid and aliphatic alcohols, such as hexanediol and/or neopentyl glycol,
where component A), according to the definition, has neither ionic nor ionogenic groups.

As component A3), polyols, in particular nonpolymeric polyols, of the specified preferred molecular weight range from 66 to 399 mol/g with up to 20 carbon atoms, such as ethylene glycol, diethylene glycol, triethylene glycol, 1,2-propanediol, 1,3-propanediol, 1,4-butanediol, 1,3-butylene - glycol, cyclohexanediol, 1,4-cyclohexanedimethanol, 1,6-hexanediol, neopentyl glycol, hydroquinone dihydroxyethyl ether, bisphenol A (2,2-bis(4-hydroxyphenyl)propane), hydrogenated bisphenol A (2,2-bis(4-hydroxycyclohexyl)propane), trimethylolpropane, trimethylolethane, glycerol, pentaerythritol and any desired mixtures thereof, can be used as desired.

Also suitable are ester diols of the specified molecular weight range, such as a-hydroxybutyl ε-hydroxycaproic acid ester, ω-hydroxyhexyl γ-hydroxybutyric acid ester, adipic acid (β-hydroxyethyl) ester or terephthalic acid bis(β-hydroxyethyl) ester.

In addition, as component A3), it is also possible to use monofunctional isocyanate-reactive hydroxyl-group-containing compounds. Examples of such monofunctional compounds are ethanol, n-butanol, ethylene glycol monobutyl ether, diethylene glycol monomethyl ether, diethylene glycol monobutyl ether, propylene glycol monomethyl ether, dipropylene glycol monomethyl ether, tripropylene glycol monomethyl ether, dipropylene glycol monopropyl ether, propylene glycol monobutyl ether, dipropylene glycol monobutyl ether, tripropylene glycol monobutyl ether, 2-ethylhexanol, 1-octanol, 1-dodecanol, 1-hexadecanol.

In one preferred embodiment of the invention, the polyurethane used according to the invention comprises less than about 10% by weight of component A3), preferably less than 5% by weight of component A3), in each case based on the total mass of the polyurethane, yet more preferably component A3) is not used for the preparation of the polyurethane.

To prepare the polyurethanes used according to the invention, one or more in particular isocyanate-reactive nonionic hydrophilizing agents are optionally used as component A4). The hydrophilizing agents used as component A4) are in particular different from components A2) and A3).

Suitable nonionically hydrophilizing compounds as component A4) are, for example, polyoxyalkylene ethers which have isocyanate-reactive groups, such as hydroxy, amino or thiol groups. Preference is given to monohydroxy-functional polyalkylene oxide polyether alcohols having, on statistical average, 5 to 70, preferably 7 to 55, ethylene oxide units per molecule, as are accessible in a manner known per se by alkoxylation of suitable starter molecules (e.g. in Ullmanns Encyclopädie der technischen Chemie [Ullmanns encyclopaedia of industrial chemistry], 4th edition, Volume 19, Verlag Chemie, Weinheim pp. 31-38). These are either pure polyethylene oxide ethers or mixed polyalkylene oxide ethers, where they contain at least 30 mol%, preferably at least 40 mol%, ethylene oxide units, based on all of the alkylene oxide units present.

Particularly preferred nonionic compounds are monofunctional mixed polyalkylene oxide polyethers which have 40 to 100 mol% ethylene oxide units and 0 to 60 mol% propylene oxide units.

Suitable starter molecules for such nonionic hydrophilizing agents are in particular saturated monoalcohols, such as methanol, ethanol, n-propanol, isopropanol, n-butanol, isobutanol, sec-butanol, the isomeric pentanols, hexanols, octanols and nonanols, n-decanol, n-dodecanol, n-tetradecanol, n-hexadecanol, n-octadecanol, cyclohexanol, the isomeric methylcyclohexanols or hydroxymethylcyclohexane, 3-ethyl-3-hydroxymethyloxetane or tetrahydrofurfuryl alcohol, diethylene glycol monoalkyl ethers, such as, for example, diethylene glycol monobutyl ether, unsaturated alcohols, such as allyl alcohol, 1,1-dimethylallyl alcohol or oleyl alcohol, aromatic alcohols, such as phenol, the isomeric cresols or methoxyphenols, araliphatic alcohols, such as benzyl alcohol, anisyl alcohol or cinnamyl alcohol, secondary monoamines, such as dimethylamine, diethylamine, dipropylamine, diisopropylamine, dibutylamine, bis(2-ethylhexyl)amine, N-methyl- and N-ethylcyclohexylamine or dicyclohexylamine, and also heterocyclic secondary amines, such as morpholine, pyrrolidine, piperidine or 1H-pyrazole. Preferred starter molecules are saturated monoalcohols of the type specified above. Particular preference is given to using diethylene glycol monobutyl ether or n-butanol as starter molecules.

Alkylene oxides suitable for the alkoxylation reaction are in particular ethylene oxide and propylene oxide, which can be used in the alkoxylation reaction in any desired order or else in a mixture.

Component B): Preferably, in step B) of the reaction of the prepolymer, a mixture of component B1) and of component B2) is reacted.

For example, organic di- or polyamines, such as, for example, 1,2-ethylenediamine, 1,2- and 1,3-diaminopropane, 1,4-diaminobutane, 1,6-diaminohexane, isophoronediamine, isomer mixture of 2,2,4- and 2,4,4-trimethylhexamethylenediamine, 2-methylpentamethylenediamine, diethylenetriamine, 4,4-diaminodicyclohexylmethane, hydrazine hydrate, and/or dimethylethylenediamine, can be used as component B1).

Moreover, compounds which, besides a primary amino group, also have secondary amino groups or, besides an amino group (primary or secondary), also have OH groups, can also be used as component B1). Examples thereof are primary/secondary amines, such as diethanolamine, 3-amino-1-methylaminopropane, 3-amino-1-ethylaminopropane, 3-amino-1-cyclohexylaminopropane, 3-amino-1-methylaminobutane, alkanolamines, such as N-aminoethylethanolamine, ethanolamine, 3-aminopropanol, neopentanolamine.

In addition, monofunctional isocyanate-reactive amine compounds can also be used as component B1), such as, for example, methylamine, ethylamine, propylamine, butylamine, octylamine, laurylamine, stearylamine, isononyloxypropylamine, dimethylamine, diethylamine, dipropylamine, dibutylamine, N-methylaminopropylamine, diethyl(methyl)aminopropylamine, morpholine, piperidine, and suitable substituted derivatives thereof, amidoamines of diprimary amines and monocarboxylic acids, monoketime of diprimary amines, primary/tertiary amines, such as N,N-dimethylaminopropylamine.

As component B1), preference is given to using 1,2-ethylenediamine, bis(4-aminocyclohexyl)methane, 1,4-diaminobutane, isophoronediamine, ethanolamine, diethanolamine and diethylenetriamine.

Component B) includes at least one component B2). Suitable anionically hydrophilizing compounds as component B2) preferably contain a sulphonic acid or sulphonate group, particularly preferably a sodium sulphonate group. Suitable anionically hydrophilizing compounds as component B2) are, in particular, the alkali metal salts of mono- and diaminosulphonic acids. Examples of such anionic hydrophilizing agents are salts of 2-(2-aminoethylamino)ethanesulphonic acid, ethylenediaminepropyl- or -butylsulphonic acid, 1,2- or 1,3-propylenediamine-β-ethylsulphonic acid or taurine. Furthermore, the salt of cyclohexylaminopropanesulphonic acid (CAPS) from WO-A 01/88006 can be used as anionic hydrophilizing agent.

Particularly preferred anionic hydrophilizing agents B2) are those which contain sulphonate groups as ionic groups and two amino groups, such as the salts of 2-(2-aminoethylamino)ethylsulphonic acid and 1,3-propylenediamine-β-ethylsulphonic acid.

The polyurethanes used according to the invention particularly preferably comprise at least one sulphonate group.

Optionally, the anionic group in component B2) may also be a carboxylate or carboxylic acid group. Component B2) is then preferably selected from diaminocarboxylic acids. However, this embodiment is less preferred since carboxylic-acid-based components B2) have to be used in higher concentrations.

For the hydrophilization, it is also possible to use mixtures of anionic hydrophilizing agents B2) and nonionic hydrophilizing agents A4).

In a preferred embodiment for the preparation of the special polyurethane dispersions, components A1) to A4) and B1) to B2) are used in the following amounts, the individual amounts always adding up to 100% by weight:
5 to 40% by weight of component A1),
55 to 90% by weight of A2),
0.5 to 20% by weight sum of components A3) and/or B1),
0.1 to 25% by weight sum of components A4) and/or B2), where, based on the total amounts of components A1) to A4) and B1) to B2), particularly preferably 0.1 to 5% by weight of anionic or potentially anionic hydrophilizing agents B2) are used.

In a particularly preferred embodiment for the preparation of the special polyurethane dispersions, components A1) to A4) and B1) to B2) are used in the following amounts, the individual amounts always adding up to 100% by weight:
5 to 35% by weight of component A1),
60 to 90% by weight of A2),
0.5 to 15% by weight sum of components A3) and/or B1),
0.1 to 15% by weight sum of components A4) and/or B2), where, based on the total amounts of components A1) to A4) and B1) to B2), particularly preferably 0.2 to 4% by weight of anionic or potentially anionic hydrophilizing agents B2) are used.

In a very particularly preferred embodiment for the preparation of the special polyurethane dispersions, components A1) to A4) and B1) to B2) are used in the following amounts, the individual amounts always adding up to 100% by weight:
10 to 30% by weight of component A1),
65 to 85% by weight of A2),
0.5 to 14% by weight sum of components A3 and/or B1),
0.1 to 13.5% by weight sum of components A4) and/or B2), where, based on the total amounts of components A1) to A4) and B1) to B2, particularly preferably 0.5 to 3.0% by weight of anionic or potentially anionic hydrophilizing agents from B2) are used.

The preparation of the polyurethane dispersions can be carried out in one or more stage(s) in homogeneous phase or, in the case of multistage reaction, sometimes in disperse phase. Following complete or partial polyaddition from A1) to A4), a dispersion, emulsification or dissolution step preferably takes place. Afterwards, a further polyaddition or modification optionally takes place in the disperse phase.

In this connection, all of the methods known from the prior art, such as, for example, prepolymer mixing process, acetone process or melt dispersion process, can be used. Preference is given to using the acetone process.

For the preparation in accordance with the acetone process, constituents A2) to A4) and the polyisocyanate component A1) for the preparation of an isocyanate-functional polyurethane prepolymer are usually initially introduced in their entirety or in part and optionally diluted with a solvent which is miscible with water but inert towards isocyanate groups, and heated to temperatures in the range from 50 to 120°C. To increase the rate of the isocyanate addition reaction, the catalysts known in polyurethane chemistry can be used.

Suitable solvents are the customary aliphatic, keto-functional solvents such as acetone, 2-butanone, which can be added not only at the start of the preparation, but optionally in parts also later on. Preference is given to acetone and 2-butanone, and particular preference is given to acetone. The addition of other solvents without isocyanate-reactive groups is also possible, but not preferred.

Any constituents of A1) to A4) not added at the start of the reaction are then metered in.

During the preparation of the polyurethane prepolymer from A1) to A4), the quantitative ratio of isocyanate groups to isocyanate-reactive groups is generally 1.05 to 3.5, preferably 1.1 to 3.0, particularly preferably 1.1 to 2.5.

The reaction of components A1) to A4) to give the prepolymer takes place partially or completely, but preferably completely. Polyurethane prepolymers which contain free isocyanate groups are thus obtained without a diluent or in solution.

In the neutralization step for the partial or complete conversion of potentially anionic groups to anionic groups, bases such as tertiary amines, e.g. trialkylamines having 1 to 12, preferably 1 to 6, carbon atoms, particularly preferably 2 to 3 carbon atoms in each alkyl radical or very particularly preferably alkali metal bases such as the corresponding hydroxides are used.

The use of organic amines is not preferred.

Neutralizing agents which can be used are preferably inorganic bases, such as aqueous ammonia solution or sodium hydroxide or potassium hydroxide.

Preference is given to sodium hydroxide and potassium hydroxide.

The quantitative amount of the bases is 50 and 125 mol%, preferably between 70 and 100 mol% of the quantitative amount of the acid groups to be neutralized. The neutralization can also take place at the same time as the dispersion by the dispersion water already comprising the neutralizing agent.

Afterwards, in a further process step, in cases where it has still not happened or has only happened partially, the resulting prepolymer is dissolved with the help of aliphatic ketones such as acetone or 2-butanone.

The reaction of components A1) to A4) to give the prepolymer takes place partially or completely, but preferably completely. In this way, polyurethane prepolymers which contain free isocyanate groups are obtained without a diluent or in solution.

During the chain extension in stage B), NH₂- and/or NH-functional components are reacted with the remaining isocyanate groups of the prepolymer. Preferably, the chain extension/termination is carried out prior to the dispersion in water.

Suitable components B) for the chain extension are, in particular, organic di- or polyamines B1), such as, for example, ethylenediamine, 1,2- and 1,3-diaminopropane, 1,4-diaminobutane, 1,6-diaminohexane, isophoronediamine, isomer mixture of 2,2,4- and 2,4,4-trimethylhexamethylenediamine, 2-methylpentamethylenediamine, diethylenetriamine, diaminodicyclohexylmethane and/or dimethylethylenediamine.

Moreover, it is also possible to use compounds B1) which, besides a primary amino group, also have secondary amino groups or, besides an amino group (primary or secondary), also have OH groups. Examples thereof are primary/secondary amines, such as diethanolamine, 3-amino-1-methylaminopropane, 3-amino-1-ethylaminopropane, 3-amino-1-cyclohexylaminopropane, 3-amino-1-methylaminobutane, alkanolamines, such as N-aminoethylethanolamine, ethanolamine, 3-aminopropanol, neopentanolamine for the chain extension and/or termination.

For the chain termination, use is usually made of amines B1) having a group which is reactive towards isocyanates, such as methylamine, ethylamine, propylamine, butylamine, octylamine, laurylamine, stearylamine, isononyloxypropylamine, dimethylamine, diethylamine, dipropylamine, dibutylamine, N-methylaminopropylamine, diethyl(methyl)aminopropylamine, morpholine, piperidine, and suitable substituted derivatives thereof, amidoamines of diprimary amines and monocarboxylic acids, monoketime of diprimary amines, primary/tertiary amines, such as N,N-dimethylaminopropylamine.

If anionic hydrophilizing agents corresponding to the definition of B2) with NH₂ or NH groups are used for the chain extension, the chain extension of the prepolymers preferably takes place before the dispersion.

The degree of chain extension, i.e. the equivalent ratio of NCO-reactive groups of the compounds used for the chain extension and chain termination to free NCO groups of the prepolymer is generally between 40 and 150%, preferably between 50 and 110%, particularly preferably between 60 and 100%.

The aminic components B1) and B2) can optionally be used in water- or solvent-diluted form in the process according to the invention individually or in mixtures, with any order of the addition being possible in principle.

If water or organic solvents are co-used as diluents, then the diluent content in the component used in B) for chain extension is preferably 40 to 95% by weight.

The dispersion preferably takes place after the chain extension. For this, the dissolved and chain-extended polyurethane polymer is optionally either introduced into the dispersion water with strong shear, such as, for example, with vigorous stirring, or, conversely, the dispersion water is stirred into the chain-extended polyurethane polymer solutions. Preferably, the water is added to the dissolved chain-extended polyurethane polymer.

The solvent still present in the dispersions after the dispersion step is then usually removed by distillation. Removal during dispersion is likewise possible.

The residual content of organic solvents in the polyurethane dispersions prepared in this way is typically less than 10% by weight, preferably less than 3% by weight, based on the total dispersion.

The pH of the aqueous polyurethane dispersions used according to the invention is typically less than 8.0, preferably less than 7.5 and is particularly preferably between 5.5 and 7.5.

The cosmetic composition according to the invention comprises preferably 0.1 to 20% by weight of the polyurethane described above and in particular 0.5 to 10% by weight, in each case based on the total weight of the composition.

The cosmetic composition according to the invention comprises preferably 0.1 to 10 % by weight more preferably 0.1 to 5 % by weight and even more preferably 0.5 to 5 % by weight of salicylic acid, in each case based on the total weight of the composition

In another embodiment, the presently claimed invention relates to a personal care product comprising at least one inventively claimed composition. In another embodiment, the presently claimed invention relates to a medical product comprising at least one inventively claimed composition.

Preferably the personal care product is selected from the group consisting of body sprays, deodorant products, detersive products, skin care products, hair care products, shaving compositions and personal cleansing products.

Within the context of the present invention, the cosmetic compositions can advantageoulsy skin cosmetic or hair cosmetic compositions.

### Skin cosmetic composition

The composition according to the invention which comprises the polyurethane described above or its aqueous dispersion should satisfy the aforementioned properties of a skincare product.. A skin cosmetic composition is defined as a cosmetic composition for the cleansing, care and protection of the skin. Within the context of the present invention, skin cosmetic compositions are skincare product, sunscreen composition, aftersun preparations, self-tarming compositions, decorative cosmetic, washing, showering and bathing preparations for use on the skin, face toners, face masks, insect repellent preparations, footcare compositions, shaving compositions, hair removal compositions, intimate care compositions, babycare compositions, deodorants and antiperspirants. Preferred skin cosmetic compositions within the context of the present invention are skincare products.

Within the context of the present invention, the skin cosmetic compositions are differentiated in particular according to their consistency: cream (viscous), lotion and milk (flowable), gels (semisolid), oils, and also balm and aqueous solutions (liquid). Depending on their formulation, the compositions according to the invention can be used, for example, as face cream, day or night cream, body lotion, face cleanser etc. It is in some instances possible that the compositions according to the invention are used as pharmaceutically active product, or comprise pharmaceutically active ingredients.

The skin cosmetic compositions may be present, for example, in the form of an aqueous or aqueous-alcoholic solution, oil-in-water, silicone-in-water, water-in-oil, water-in-silicone emulsion, multiple emulsion, such as, oil-in-water-in-oil, water-in-oil-in-water emulsion, polymer stabilized emulsion (so called hydrodispersion), solids satbilized emulsion (also called pickering emulsion), PIT emulsion

Ingredients for the Cosmetic Compositions According to the Invention

### Emulsifiers

The cosmetic compositions can comprise one or more emulsifiers or surface-active agents.

Thus, in particular oil-in-water emulsions (O/W) according to the invention comprise preferably at least one emulsifier with an HLB value of > 7 and, if appropriate, a coemulsifier.

O/W emulsifiers can advantageously be selected from the group of nonionic, anionic, cationic or amphoteric emulsifiers.

The nonionic emulsifiers include:
a) partial fatty acid esters and fatty acid esters of polyhydric alcohols and ethoxylated derivatives thereof
b) ethoxylated fatty alcohols and fatty acids
c) ethoxylated fatty amines, fatty acid amides, fatty acid alkanolamides
d) alkylphenol polyglycol ethers (e.g. Triton® X)
e) ethoxylated fatty alcohol ethers.

Particularly advantageous nonionic O/W emulsifiers are ethoxylated fatty alcohols or fatty acids, preferably PEG-100 stearate, PEG-40 stearate, PEG-50 stearate, ceteareth-20, ceteth-20, steareth-20, ceteareth-12, ceteth-12, steareth-12, esters of mono-, oligo- or polysaccharides with fatty acids, preferably cetearyl glucoside, methylglucose distearate, glyceryl monostearates (self-emulsifying), sorbitan esters, such as, for example, sorbitan stearates (Tween® 20 and Tween® 60 from Uniqema), sorbitan palmitates (Span® 40, Uniqema), glyceryl stearyl citrates, sucrose esters, such as, for example, sucrose stearates, PEG-20 methyl glucose sesquistearate), dicarboxylic acid esters of fatty alcohol (dimyristyl tartrate).

Advantageous anionic emulsifiers are soaps (e.g. sodium or triethanolamine salts of stearic acid or palmitic acid), esters of citric acid, such as glyceryl stearate citrate, fatty alcohol sulphates, and also mono-, di- and trialkyl phosphoric acid esters and ethoxylates thereof.

The cationic emulsifiers include quaternary ammonium compounds with a long-chain aliphatic radical, e.g. distearyl dimonium chloride.

The amphoteric emulsifiers include:
a) alkylaminoalkane carboxylic acids
b) betaines, sulphobetaines
c) imidazoline derivatives.

Furthermore, there are naturally occurring emulsifiers, which include beeswax, wool wax, lecithin and sterols.

Suitable coemulsifiers for the O/W emulsions according to the invention which can be used are fatty alcohols having 8 to 30 carbon atoms, monoglycerol esters of saturated or unsaturated, branched or unbranched alkanecarboxylic acids with a chain length of from 8 to 24 carbon atoms, in particular 12 to 18 carbon atoms, propylene glycol esters of saturated or unsaturated, branched or unbranched alkanecarboxylic acids with a chain length of from 8 to 24 carbon atoms, in particular 12 to 18 carbon atoms, and also sorbitan esters of saturated or unsaturated, branched or unbranched alkanecarboxylic acids with a chain length of from 8 to 24 carbon atoms, in particular 12 to 18 carbon atoms.

Particularly advantageous coemulsifiers are glyceryl monostearate, glyceryl monooleate, diglyceryl monostearate, sorbitan monoisostearate, sucrose distearate, cetyl alcohol, stearyl alcohol, behenyl alcohol, isobehenyl alcohol and polyethylene glycol(2) stearyl ether (steareth-2).

Within the context of the present invention, it may be advantageous to use further emulsifiers. Thus, for example, the water resistance of the preparations according to the invention can be increased. Suitable emulsifiers are, for example, alkylmethicone copolyols and alkyldimethicone copolyols, in particular cetyldimethicone copolyol, laurylmethicone copolyol, W/O emulsifiers, such as sorbitan stearate, glyceryl stearate, glycerol stearate, sorbitan oleate, lecithin, glyceryl isostearate, polyglyceryl-3 oleate, polyglyceryl-3 diisostearate, PEG-7-hydrogenated castor oil, polyglyceryl-4 isostearate, acrylate/C₁₀₋₃₀-alkyl acrylate crosspolymer, sorbitan isostearate, poloxamer 101, polyglyceryl-2 dipolyhydroxystearate, polyglyceryl-3 diisostearate, polyglyceryl-4 dipolyhydroxystearate, PEG-30 dipolyhydroxystearate, diisostearoyl polyglyceryl-3 diisostearate, glycol distearate and polyglyceryl-3 dipolyhydroxystearate.

The cosmetic compositions according to the invention, can advantageously comprise thickeners of the water phase. Advantageous thickeners are:
- Crosslinked or uncrosslinked acrylic acid or methacrylic acid homopolymers or copolymers. These include crosslinked homopolymers of methacrylic acid or acrylic acid, copolymers of acrylic acid and/or methacrylic acid and monomers which are derived from other acrylic or vinyl monomers, such as C10-30 alkyl acrylates, C10-30-alkyl methacrylates and vinyl acetate and vinylpyrrolidones.
- Thickening polymers of natural origin, for example based on cellulose, guar gum, xanthan, scleroglucan, gellan gum, rhamsan and karaya gum, alginates, maltodextrin, starch and its derivatives, carob seed flour, hyaluronic acid, carrageenan.
- Nonionic, anionic, cationic or amphoteric associative polymers, e.g. based on polyethylene glycols and their derivatives, or polyurethanes.
- Crosslinked or uncrosslinked homopolymers or copolymers based on acrylamide or methacrylamide, such as homopolymers of 2-acrylamido-2-methylpropanesulphonic acid, copolymers of acrylamide or methacrylamide and methacryloyloxyethyltrimethylammonium chloride or copolymers of acrylamide and 2-acrylamido-2-methylpropanesulphonic acid.

Particularly advantageous thickeners are thickening polymers of natural origin, crosslinked acrylic acid or methacrylic acid homopolymers or copolymers and crosslinked copolymers of 2-acrylamido-2-methylpropanesulphonic acid.

Very particularly advantageous thickeners are xanthan gum, such as the products supplied under the names Keltrol® and Kelza® by CP Kelco or the products from RHODIA with the name Rhodopol, and guar gum, such as the products available under the name Jaguar® HP105 from RHODIA.

Very particularly advantageous thickeners are crosslinked homopolymers of methacrylic acid or acrylic acid which are commercially available from Lubrizol under the names Carbopol® 940, Carbopol® 941, Carbopol® 980, Carbopol® 981, Carbopol® ETD 2001, Carbopol® EDT 2050, Carbopol® 2984, Carbopol® 5984 and Carbopol® Ultrez 10, from 3V under the names Synthalen® K, Synthalen® L and Synthalen® MS.

Very particularly advantageous thickeners are crosslinked polymers of acrylic acid or methacrylic acid and a C₁₀₋₃₀-alkyl acrylate or C₁₀₋₃₀-alkyl methacrylate and copolymers of acrylic acid or methacrylic acid and vinylpyrrolidone. Such copolymers are commercially available, for example, from Lubrizol under the names Carbopol® 1342, Carbopol® 1382, Pemulen® TR1 or Pemulen® TR2 and from ISP under the names Ultrathix P-100 (INCI: Acrylic Acid/VP Crosspolymer).

Very particular advantageous thickeners are crosslinked copolymers of 2-acrylamido-2-methylpropanesulphonic acid. Such copolymers are available, for example, from Clariant under the names Aristoflex® AVC (INCI: Ammonium Acryloyldimethyltaurate/VP Copolymer).

These thickeners are generally present in a concentration of from about 0% to 2% by weight, preferably 0% to 1% by weight, based on the total weight of the composition according to the invention.

Further compositions according to the invention may be water-in-oil or water-in-silicone emulsions. Preference is given to water-in-oil (W/O) or water-in-silicone emulsions (W/Si) which comprise one or more silicone emulsifiers (W/S) with an HLB value of ≤ 8 or one or more W/O emulsifiers with an HLB value of < 7 and optionally one or more O/W emulsifiers with an HLB value of > 10.

The silicone emulsifiers can advantageously be selected from the group comprising alkyldimethicone copolyols, such as, for example, cetyl PEG/PPG 10/1 dimethicone copolyol (ABIL® EM 90 from Goldschmidt AG) or lauryl PEG/PPG- 18/18 dimethicones (Dow Corning® 5200 from Dow Corning Ltd.) and dimethicone copolyols, such as, for example, PEG-10 dimethicones (KF-6017 from Shin Etsu), PEG/PPG- 18/18 dimethicones (Dow Corning 5225C from Dow Corning Ltd.) or PEG/PPG-19/19 dimethicones (Dow Corning BY-11 030 from Dow Corning Ltd.) or trimethylsilylamodimethicones.

The W/O emulsifiers with an HLB value of < 7 can advantageously be selected from the following group: fatty alcohols having 8 to 30 carbon atoms, monoglycerol esters of saturated and/or unsaturated, branched and/or unbranched alkanecarboxylic acids of chain length of from 8 to 24, in particular 12-18 carbon atoms, diglycerol esters of saturated and/or unsaturated, branched and/or unbranched alkanecarboxylic acids of chain length from 8 to 24, in particular 12-18, carbon atoms, monoglycerol ethers of saturated and/or unsaturated, branched and/or unbranched alcohols of chain length of from 8 to 24, in particular 12-18, carbon atoms, diglycerol ethers of saturated and/or unsaturated, branched and/or unbranched alcohols of chain length from 8 to 24, in particular 12-18, carbon atoms, propylene glycol esters of saturated and/or unsaturated, branched and/or unbranched alkanecarboxylic acids of chain length from 8 to 24, in particular 12-18, carbon atoms, and also sorbitan esters of saturated and/or unsaturated, branched and/or unbranched alkanecarboxylic acids of chain length from 8 to 24, in particular 12-18, carbon atoms.

Particularly advantageous W/O emulsifiers are: glyceryl monostearate, glyceryl monoisostearate, glyceryl monomyristate, glyceryl monooleate, diglyceryl monostearate, diglyceryl monoisostearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, sucrose distearate, cetyl alcohol, stearyl alcohol, arachidyl alcohol, behenyl alcohol, isobehenyl alcohol, selachyl alcohol, chimyl alcohol, polyethylene glycol(2) stearyl ether (steareth-2), glyceryl monolaurate, glyceryl monocaprate and glyceryl monocaprylate.

Further possible W/O emulsifiers are selected from the group of the compounds polyglyceryl-2 dipolyhydroxystearate, PEG-30 dipolyhydroxystearate, cetyldimethicone copolyol and polyglyceryl-3 diisostearate.

The O/W emulsifiers with an HLB value of > 10 can advantageously be selected from the group comprising lecithin, trilaureth-4 phosphate, polysorbate-20, polysorbate-60, PEG-22 dodecyl glycol copolymer, sucrose stearate and sucrose laurate.

An oil thickener can advantageously be used for stabilizing the W/O emulsion according to the invention against sedimentation or flocculation of the water droplets.

Particularly advantageous oil thickeners are organomodified clays, such as organomodified bentonites (Bentone® 34 from Rheox), organomodified hectorites (Bentone® 27 and Bentone® 38 from Rheox) or organomodified montmorillonite, hydrophobic pyrogenic silica, where the silanol groups are substituted by trimethylsiloxy groups (AEROSIL® R812 from Degussa) or with dimethylsiloxy groups or polydimethylsiloxane (AEROSIL® R972, AEROSIL® R974 from Degussa, CAB-O-SIL® TS-610, "CAB-O-SIL® TS-720 from Cabot), magnesium or aluminium stearate, or styrene copolymers, such as, for example, styrene-butadiene-styrene, styrene-isopropene-styrene, styrene-ethylene/butene-styrene or styrene-ethylene/propene-styrene.

The thickener for the fatty phase can be present in an amount of from 0.1 to 5% by weight, based on the total weight of the emulsion, and better 0.4 to 3% by weight.

The aqueous phase can also comprise stabilizers. The stabilizer can be, for example, sodium chloride, magnesium chloride or magnesium sulphate and mixtures thereof.

### Oils, Fats, Waxes

Oils can be used in W/O, W/Si and O/W emulsions.

If present, the fatty phase of the composition according to the invention can comprise one non-volatile oil and/or volatile oils and waxes. The O/W composition comprises advantageously 0.01 to 45% by weight of oils, based on the total weight of the composition, and particularly advantageously 0.01 to 20% by weight of oils. The W/O or W/Si composition advantageously comprises at least 20% by weight of oils, based on the total weight of the composition.

The non-volatile oil is advantageously selected from the group of mineral, animal, vegetable or synthetic origin, polar or nonpolar oils and mixtures thereof.

The lipid phase of the cosmetic or dermatological emulsions according to the invention can advantageously be selected from the following group of substances:
mineral oils, mineral waxes, polar oils, such as triglycerides of capric acid or of caprylic acid, also natural oils, such as, for example, castor oil, fats, waxes and other natural and synthetic fatty bodies, preferably esters of fatty acids with alcohols of low carbon number, e.g. with isopropanol, propylene glycol or glycerol, or esters of fatty alcohols with alkanoic acids of low carbon number or with fatty acids; alkyl benzoates; silicone oils, such as dimethylpolysiloxanes, diethylpolysiloxanes, diphenylpolysiloxanes, and mixed forms thereof.

The polar oils are advantageously selected from the group:
a) esters of saturated and/or unsaturated, branched and/or unbranched alkanecarboxylic acids of chain length from 3 to 30 carbon atoms and saturated and/or unsaturated, branched and/or unbranched alcohols of chain length from 3 to 30 carbon atoms,
b) esters of aromatic carboxylic acids and saturated and/or unsaturated, branched and/or unbranched alcohols of chain length from 3 to 30 carbon atoms.
   Such ester oils can then advantageously be selected from the group:
   isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, isotridecyl isononanoate, 2-ethylhexyl palmitate, 2-ethylhexyl laurate, 2-ethylhexyl isostearate, 2-hexyldecyl stearate, 2-octyldodecyl palmitate, 2-ethylhexyl cocoate, oleyl oleate, oleyl erucate, erucyl oleate, erucyl erucate, dicaprylyl carbonate (Cetiol® CC) and cocoglycerides (Myritol® 331), and also synthetic, semisynthetic and natural mixtures of such esters, e.g. jojoba oil.
c) alkyl benzoates C12-15-alkyl benzoate (Finsolv® TN from Finetex) or 2-phenylethyl benzoate (X-Tend® 226 from ISP)
d) lecithins and the fatty acid triglycerides, namely the triglycerol esters of saturated and/or unsaturated, branched and/or unbranched alkanecarboxylic acids of chain length from 8 to 24, in particular 12 to 18 carbon atoms. For example, the fatty acid triglycerides can be selected from the group of cocoglyceride, olive oil, sunflower oil, soybean oil, peanut oil, rapeseed oil, almond oil, palm oil, coconut oil, castor oil, wheat germ oil, grapeseed oil, safflower oil, evening primrose oil, macadamia nut oil, apricot kernel oil, avocado oil and the like.
e) dialkyl ethers and dialkyl carbonates, e.g. dicaprylyl ether (Cetiol® OE from Cognis) and/or dicaprylyl carbonate (for example Cetiol® CC from Cognis) are advantageous
f) saturated or unsaturated, branched or unbranched alcohols, such as, for example, octyldodecanol.

The non-volatile oil can likewise advantageously also be a nonpolar oil which is selected from the group of branched and unbranched hydrocarbons, in particular mineral oil, vaseline oil, paraffin oil, squalane and squalene, polyolefins, for example polydecenes, hydrogenated polyisobutenes, C13-16 isoparaffin and isohexadecane.

The nonpolar non-volatiile oil can be selected among the non-volatile silicone oils.

Of the non-volatile silicone oils, the polydimethylsiloxanes (PDMS), which are optionally phenylated, such as phenyltrimethicone, or are optionally substituted with aliphatic and/or aromatic groups or with functional groups, for example hydroxyl groups, thiol groups and/or amino groups; polysiloxanes modified with fatty acids, fatty alcohols or polyoxyalkylenes and mixtures thereof can be given.

Particularly advantageous oils are 2-ethylhexyl isostearate, octyldodecanol, isotridecyl isononanoate, isoeicosane, 2-ethylhexyl cocoate, C12-15 alkyl benzoate, caprylic/capric triglyceride, dicaprylyl ether, mineral oil, dicaprylyl carbonate, cocoglycerides, butylene glycol dicaprylate/dicaprate, hydrogenated polyisobutenes, cetaryl isononanoates, isodecyl neopentanoates, squalane, C13-16 isoparaffin.

The composition according to the invention can also comprise a wax.

Within the context of the present specification, a wax is defined as a lipophilic fatty substance which is solid at room temperature (25°C) and exhibits a reversible solid/liquid change in state at a melting temperature between 30°C and 200°C. Above the melting point, the wax becomes low viscosity and miscible with oils.

The wax is advantageously selected from the groups of natural waxes, such as, for example, cotton wax, carnauba wax, candelilla wax, esparto wax, Japan wax, Montan wax, sugarcane wax, beeswax, wool wax, shellac, microwaxes, ceresine, ozokerite, ouricury wax, cork fibre wax, lignite waxes, berry wax, shea butter or synthetic waxes, such as paraffin waxes, polyethylene waxes, waxes produced by Fischer-Tropsch synthesis, hydrogenated oils, fatty acid esters and glycerides which are solid at 25°C, silicone waxes and derivatives (alkyl derivatives, alkoxy derivatives, and/or esters of polymethylsiloxane) and mixtures thereof. The waxes can be present in the form of stable dispersions of colloidal wax particles which can be prepared by known processes, for example as in "Microemulsions Theory and Practice", L.M. Prince Ed., Academic Press (1977), pages 21-32.

Waxes may be present in amounts of from 0 to 10% by weight, based on the total weight of the composition, and preferably 0 to 5% by weight.

The cosmetic composition according to the invention can also comprise a volatile oil which is selected from the group of volatile hydrocarbon oils, siliconized oils or fluorinated oils.

The volatile oil can be present in an amount of from 0 to 25% by weight, based on the total weight of the emulsion, preferably 0 to 20% by weight and even more preferably 0 to 15% by weight.

Within the context of the present specification, a volatile oil is an oil which, upon contact with the skin at room temperature and atmospheric pressure, evaporates in less than one hour. The volatile oil is liquid at room temperature and, at room temperature and atmospheric pressure, has a vapour pressure of from 0.13 to 40 000 Pa (10⁻³ to 300 mm Hg), preferably 1.3 to 13 000 Pa (0.01 to 100 mmHg) and particularly preferably 1.3 to 1300 Pa (0.01 to 10 mmHg) and a boiling point of from 150 to 260°C and preferably 170 to 250°C.

A hydrocarbon oil is understood as meaning an oil which is formed from carbon atoms and hydrogen atoms and optionally oxygen atoms or nitrogen atoms and contains no silicon atoms or fluorine atoms, where it may also consist of carbon atoms and hydrogen atoms; however, it can also contain ester groups, ether groups, amino groups or amide groups.

A siliconized oil is understood as meaning an oil which contains at least one silicon atom and in particular Si-O groups.

A fluorinated oil is to be understood as meaning an oil which contains at least one fluorine atom.

The volatile hydrocarbon oil according to the invention can be selected from the hydrocarbon oils with a flash point of from 40 to 102°C, preferably 40 to 55°C and even more preferably 40 to 50°C.

For example, the volatile hydrocarbon oils are those with 8 to 16 carbon atoms and mixtures thereof, in particular branched C₈₋₁₆-alkanes, such as the isoalkanes (which are also referred to as isoparaffins) with 8 to 16 carbon atoms, isododecane, isodecane, isohexadecane and, for example, the oils which are supplied under the tradenames Isopars® or Permetyls®; and the branched C₈₋₁₆-esters, such as isohexyl neopentanoate and mixtures thereof.

The volatile hydrocarbon oils such as isododecane, isodecane and isohexadecane are particularly advantageous.

The volatile siliconized oil according to the invention can be selected from the siliconized oils with a flash point of from 40 to 102°C, preferably a flash point above 55°C and at most 95°C and particularly preferably in the range from 65 to 95°C.

For example, the volatile siliconized oils are straight-chain or cyclic silicone oils having 2 to 7 silicon atoms, where these silicones optionally contain alkyl or alkoxy groups having 1 to 10 carbon atoms.

The volatile siliconized oils such as octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane and mixtures thereof are particularly advantageous.

The volatile fluorinated oil generally has no flash point.

For example, the volatile fluorinated oils are nonafluoroethoxybutane, nonafluoromethoxybutane, decafluoropentane, tetradecafluorohexane, dodecafluoropentane and mixtures thereof.

### Surfactants

The cosmetic compositions according to the invention can also comprise surfactants which are selected from the group of anionic, cationic, nonionic and/or amphoteric surfactants.

Advantageous anionic surfactants within the context of the present invention are:
acylamino acids and salts thereof; such as acylglutamates, in particular sodium acyl glutamate and sarcosinates, for example myristoyl sarcosine, TEA lauroyl sarcosinate, sodium lauroyl sarcosinate and sodium cocoyl sarcosinate;
sulphonic acids and salts thereof, such as acyl isethionates, for example sodium or anmonium cocoyl isethionate, sulphosuccinates, for example dioctyl sodium sulphosuccinate, disodium laureth sulphosuccinate, disodium lauryl sulphosuccinate and disodium undecylenamido MEA sulphosuccinate, disodium PEG-5 lauryl citrate sulphosuccinate and derivatives;
sulphuric acid esters, such as alkyl ether sulphate, for example sodium, ammonium, magnesium, MIPA, TIPA laureth sulphate, sodium myreth sulphate and sodium CI2-13 pareth sulphate, and alkyl sulphates, for example sodium, ammonium and TEA lauryl sulphate;
taurates, for example sodium lauroyl taurate and sodium methylcocoyl taurate or ether carboxylic acids, for example sodium laureth-13 carboxylate and sodium PEG-6 cocamide carboxylate, sodium PEG-7 olive oil carboxylate;
phosphoric acid esters and salts, such as, for example, DEA oleth-10 phosphate and dilaureth-4 phosphate;
alkylsulphonates, for example sodium coconut monoglyceride sulphate, sodium CI2 to C14-olefinsulphonate, sodium lauryl sulphoacetate and magnesium PEG-3 cocamidosulphate;
acyl glutamates, such as di-TEA palmitoyl aspartate and sodium caprylic/capric glutamate,
acyl peptides, for example palmitoyl hydrolysed milk protein, sodium cocoyl hydrolysed soya protein and sodium/potassium cocoyl hydrolysed collagen;
carboxylic acids and derivatives, such as, for example, lauric acid, aluminium stearate, magnesium alkanolate and zinc undecylenate, ester carboxylic acids, for example calcium stearoyllactylate, laureth-6 citrate and sodium PEG-4 lauramide carboxylate; and
alkylarylsulphonates.

Within the context of the present invention, advantageous cationic surfactants are quaternary surfactants. Quaternary surfactants contain at least one N atom which is covalently bonded to 4 alkyl or aryl groups. Alkylbetaine, alkylamidopropylbetaine and alkylamidopropylhydroxysultaine, for example, are advantageous.

Further advantageous cationic surfactants within the context of the present invention are also alkylamines, alkylimidazoles and ethoxylated amines and in particular salts thereof.

Advantageous amphoteric surfactants within the context of the present invention are acyl/dialkylethylenediamines, for example sodium acyl amphoacetate, disodium acyl amphodipropionate, disodium alkyl amphodiacetate, sodium acyl amphohydroxypropylsulphonate, disodium acyl amphodiacetate, sodium acyl amphopropionate, and N-coconut fatty acid amidoethyl N-hydroxyethylglycinate sodium salts.

Further advantageous amphoteric surfactants are N-alkylamino acids, for example aminopropylalkylglutamide, alkylaminopropionic acid, sodium alkylimidodipropionate and lauroamphocarboxyglycinate.

Advantageous active nonionic surfactants within the context of the present invention are alkanolamides, such as cocamides MEA/DEA/MIPA, esters which are formed by esterification of carboxylic acids with ethylene oxide, glyceryl, sorbitan or other alcohols, ethers, for example ethoxylated alcohols, ethoxylated lanoline, ethoxylated polysiloxanes, propoxylated POE ethers, alkyl polyglycosides, such as lauryl glucoside, decyl glycoside and cocoglycoside, glycosides with an HLB value of at least 20 (e.g. Belsil®SPG 128V from Wacker).

Further advantageous nonionic surfactants are alcohols and amine oxides, such as cocoamidopropylamine oxide.

Among the alkyl ether sulphates, preference is given in particular to sodium alkyl ether sulphates based on di-or triethoxylated lauryl and myristyl alcoho!. They are significantly superior to the alkyl sulphates with regard to the insensitivity towards water hardness, the ability to be thickened, the solubility at low temperature and in particular the skin and mucosa compatibility. Lauryl ether sulphate has better foam properties than myristyl ether sulphate, but is inferior to this in terms of mildness.

Alkyl ether carboxylates are types of the mildest surfactants in general, but exhibit poor foam and viscosity behaviour. They are often used in combination with alkyl ether sulphates and amphoteric surfactants.

Sulphosuccinic acid esters (sulphosuccinates) are mild and readily foaming surfactants, but on account of their poor ability to be thickened, are preferably used only together with other anionic and amphoteric surfactants and, on account of their low hydrolysis stability, are used preferably only in neutral or well buffered products.

Amidopropylbetaines have excellent skin and eye mucosa compatibility. In combination with other surfactants, their mildness can be improved synergistically. Preference is given to the use of cocamidopropylbetaine.

Amphoacetates/amphodiacetates have, as amphoteric surfactants, very good skin and mucosa compatibility and can have a conditioning effect and/or increase the care effect of supplements. Like the betaines, they are used for optimizing alkyl ether sulphate formulations. Sodium cocoamphoacetate and disodium cocoamphodiacetate are most preferred.

Alkyl polyglycosides are mild, have good universal properties, but are weakly foaming. For this reason, they are preferably used in combinations with anionic surfactants.

### Solvents

The cosmetic acceptable medium of the cosmetic composition according to the invention comprises water and optionally a cosmetically suitable water-miscible organic solvent.

The water used in the cosmetic composition according to the invention may be a blossom water, pure demineralized water, mineral water, thermal water and/or seawater.

In the case of an O/W composition, the water fraction can be in the range from 40 to 95% by weight, preferably in the range from 50 to 90% by weight, very particularly in the range from 60 to 80% by weight, based on the total weight of the composition. In the case of a W/O composition, the water fraction is in the range from 0 to 60% by weight, preferably in the range from 10 to 50% by weight, very preferably in the range from 30 to 50% by weight, based on the total weight of the composition.

The preferred solvents are, for example, the aliphatic alcohols with C1-4 carbon atoms, such as ethanol and isopropanol; polyol and derivatives thereof, such as propylene glycol, dipropylene glycol, butylene-1,3 glycol, polypropylene glycol, glycol ethers such as alkyl (CI-4) ethers of mono-, di- or tripropylene glycol or mono-, di- or triethylene glycol, and mixtures thereof.

The quantitative fraction of the solvent or solvents in the composition according to the invention can be, for example, in the range from 0 to 25% by weight and preferably 0 to 15% by weight, based on the total weight of the composition.

The cosmetic composition according to the invention can additionally comprise additives which are customary in cosmetics, such as antioxidants, photoprotective agents and/or other auxiliaries and additives, such as, for example, emulsifiers, interface-active substances, antifoams, thickeners, surfactants, active ingredients, humectants, filler, UV filters, film formers, solvents, coalescing agents, aroma substances, odour absorbers, perfumes, gel formers and/or other polymer dispersions, such as, for example, dispersions based on polyacrylates or polyurethane, pigments, dyes, flow agents and/or thixotropic agents, suppleness agents, softeners, preservatives. The amounts of the various additives are known to the person skilled in the art for the range to be used and are, for example, in the range from 0 to 25% by weight, based on the total weight of the composition.

### Sensory Additives

The cosmetic composition according to the invention can also comprise sensory additives. Sensory additives are to be understood as meaning colourless or white, mineral or synthetic, lamellar, spherical or elongated inert particles or a nonparticulate sensory additive which, for example, further improve the sensory properties of the formulations and, for example, leave behind a velvety or silky skin feel.

The sensory additives can be present in the composition according to the invention, for example, in an amount of from 0 to 10% by weight, based on the total weight of the composition, and preferably from 0 to 7%.

Advantageous particulate sensory additives within the context of the present invention are talc, mica, silicon dioxide, kaolin, starch and derivatives thereof (for example tapioca starch, distarch phosphate, aluminium and sodium starch octenyl succinate and the like), pyrogenic silica, pigments which have neither primarily a UV-filter effect nor colouring effect (such as e.g. boron nitride etc.), boron nitride, calcium carbonate, dicalcium phosphate, magnesium carbonate, magnesium hydrogencarbonate, hydroxyapatites, microcrystalline cellulose, powders of synthetic polymers, such as polyamides (for example the polymers available under the trade name "Nylon®"), polyethylene, poly-β-alanine, polytetrafluoroethylene ("Teflon®"), polyacrylate, polyurethane, lauroyl-lysine, silicone resin (for example the polymers available under the trade name "Tospearl®" from Kobo Products Inc.), hollow particles of polyvinylidene/acrylonitriles (Expancel® from Akzo Nobel) or hollow particles of silicon oxide (Silica Beads® from MAPRECOS).

Advantageous nonparticulate sensory additives can be selected from the group of dimethiconols (e.g. Dow Corning 1503 Fluid from Dow Corning Ltd.), silicone copolymers (e.g. divinyldimethicone/dimethicone copolymer, Dow Corning HMW 2220 from Dow Corning Ltd.) or silicone elasters (e.g. dimethicone crosspolymer, Dow Corning 9040 Silicone Elastomer Blend from Dow Corning Ltd.).

### Sunscreen Filters

The cosmetic composition according to the invention can optionally also comprise sunscreen filters, where the total amount of the sunscreen filter is 0% by weight to 30% by weight, 0% by weight to 20% by weight, particularly advantageously 0% by weight to 10% by weight, based on the total weight of the composition according to the invention. The sunscreen filters (or UV filters) can in particular be selected from the organic filters, the physical filters and mixtures thereof.

The composition according to the invention can comprise UV-A filters, UV-B filters or broadband filters. The UV filters used can be oil-soluble or water-soluble. The list of specified UV filters below is of course not limiting.

Examples of the UV-B filters are:
- (1) salicylic acid derivatives, particularly homomenthyl salicylate, octyl salicylate and 4-isopropylbenzyl salicylate;
- (2) cinnamic acid derivatives, in particular 2-ethylhexyl p-methoxycinnamate, which is available from Givaudan under the name Parsol MCX® and isopentyl 4-methoxycinnamate;
- (3) liquid β,β'-diphenylacrylate derivatives, in particular 2-ethylhexyl α,β'-diphenylacrylate or octocrylene, which is available from BASF under the name UVINUL N539®;
- (4) p-aminobenzoic acid derivatives, in particular 2-ethylhexyl 4-(dimethylamino)benzoate, amyl 4-(dimethylamino)benzoate;
- (5) 3-benzylidenecamphor derivatives, in particular 3-(4-methylbenzylidene)camphor which is commercially available from Merck under the name EUSOLEX 6300®, 3-benzylidenecamphor, benzylidenecamphor sulphonic acid and polyacrylamidomethyl-benzylidenecamphor;
- (6) 2-phenylbenzimidazole-5-sulphonic acid, which is available under the name EUSOLEX 232® from Merck;
- (7) 1,3,5-triazine derivatives, in particular: - 2,4,6-tris[p-(2'-ethylhexyl-1'-oxycarbonyl)anilino]-1,3,5-triazine, which is supplied by BASF under the name UVINUL T150®, and - dioctylbutamidotriazone, which is supplied by Sigma 3V under the name UVASORB HEB®;
- (8) esters of benzalmalonic acid, in particular di(2-ethylhexyl) 4-methoxybenzalmalonate and 3-(4-(2,2-bisethoxycarbonylvinyl)-phenoxy)propenyl)methoxysiloxane/dimethylsiloxane copolymer, which is available from Roche Vitamines under the name Parsol® SLX; and
- (9) the mixtures of these filters.

Examples of UV-A filters are:
- (1) dibenzoylmethane derivatives, particularly 4-(t-butyl)-4'-methoxydibenzoylmethane, which is supplied by Givaudan under the name PARSOL 1789® and 1-phenyl-3-(4'-isopropylphenyl)propane-1,3-dione;
- (2) benzene-1,4-[di(3-methylidenecamphor-10-sulphonic acid)], optionally completely or partially neutralized, commercially available under the name MEXORYL SX® from Chimex.
- (3) hexyl 2-(4'-diethylamino-2'-hydroxybenzoyl)benzoate (also aminobenzophenone);
- (4) silane derivatives or polyorganosiloxanes with benzophenone groups;
- (5) anthranilates, particularly menthyl anthranilate, which is supplied by Symrise under the name NEO HELIOPAN MA®;
- (6) compounds which contain at least two benzoazolyl groups or at least one benzodiazolyl group per molecule, in particular 1,4-bis-benzimidazolylphenylene-3,3',5,5'-tetrasulphonic acid and its salts, which are commercially available from Symrise;
- (7) silicon derivatives of benzimidazolylbenzazoles, which are N-substituted, or of benzofuranylbenzazoles, in particular: - 2-[1-[3-[1,3,3,3-tetramethyl-1-[(trimethylsilyl)oxy]disiloxanyl]propyl]-1H-benzimidazol-2-yl]benzoxazole; - 2-[1-[3-[1,3,3,3-tetramethyl-1-[(trimethylsilyl)oxy]disiloxanyl]propyl]-1H-benzimidazol-2-yl]benzothiazole; - 2-[1-(3-trimethylsilanylpropyl)-1H-benzimidazol-2-yl]benzoxazole; - 6-methoxy-1,1'-bis(3-trimethylsilanylpropyl)1H,1'H-[2,2']dibenzimidazolylbenzoxazole; - 2-[1-(3-trimethylsilanylpropyl)-1H-benzimidazol-2-yl]benzothiazole; which are described in the patent application EP-A-1 028 120;
- (8) triazine derivatives, in particular 2,4-bis[5-1(dimethylpropyl)benzoxazol-2-yl-(4-phenyl)imino]-6-(2-ethylhexyl)imino-1,3,5-triazine, which is supplied by 3V under the name Uvasorb®K2A; and
- (9) mixtures thereof.

Examples of broadband filters are:
- (1) benzophenone derivatives, for example - 2,4-dihydroxybenzophenone (benzophenone-1); - 2,2',4,4'-tetrahydroxybenzophenone (benzophenone-2); - 2-hydroxy-4-methoxybenzophenone (benzophenone-3), available from BASF under the name UNIVNUL M40®; - 2-hydroxy-4-methoxybenzophenone-5-sulphonic acid (benzophenone-4), and its sulphonate form (benzophenone-5), commercially available from BASF under the name UVINUL MS40®; - 2,2'-dihydroxy-4,4'-dimethoxybenzophenone (benzophenone-6-); - 5-chloro-2-hydroxybenzophenone (benzophenone-7-); - 2,2'-dihydroxy-4-methoxybenzophenone (benzophenone-8); - the disodium salt of 2,2'-dihydroxy-4,4'-dimethoxybenzophenone-5,5'-disulphonic acid (benzophenone-9-); - 2-hydroxy-4-methoxy-4'-methylbenzophenone (benzophenone-10); - benzophenone-11; - 2-hydroxy-4-(octyloxy)benzophenone (benzophenone-12).
- (2) triazine derivatives, in particular 2,4-bis{[4-2-ethylhexyloxy)-2-hydroxy]-phenyl}-6-(4-methoxyphenyl)-1,3,5-triazine, which is supplied by Ciba Geigy under the name TINOSORB S®, and 2,2'-methylenebis[6-(2H-benzotriazol-2-yl)4-(1,1,3,3-tetramethylbutyl)phenol], which is available from Ciba Geigy under the name TINOSORB M®; and
- (3) 2-(1H-benzotriazol-2-yl)-4-methyl-6-[2-methyl-3-[1,3,3,3-tetramethyl-1-[(trimethylsilyl)oxy]disiloxanyl]propyl]phenol with the INCI name Drometrizole Trisiloxane.

It is also possible to use a mixture of two or more filters and a mixture of UV-B filters, UV-A filters and broadband filters, and also mixtures with physical filters.

Of the physical filters, the sulphates of barium, oxides of titanium (titanium dioxide, amorphous or crystalline in the form of rutile and/or anatase), of zinc, of iron, of zirconium, of cerium, silicon, manganese or mixtures thereof may be given. The metal oxides can be present in particle form with a size in the micrometre range or nanometre range (nanopigments). The average particle sizes for the nanopigments are, for example, 5 to 100 nm.

### Humectants

The cosmetic compositions according to the invention can furthermore comprise humectants.

Particularly advantageous humectants or moisturizers within the context of the present invention are, for example, glycerol, polyglycerol, sorbitol, dimethyl isosorbide, lactic acid and/or lactates, in particular sodium lactate, butylene glycol, propylene glycol, biosaccaride gum-1, glycine soya, hydroxyethylurea, ethylhexyloxyglycerol, pyrrolidonecarboxylic acid and urea. In addition, it is especially advantageous to use polymeric "moisturizers" from the group of water-soluble and/or water-swellable and/or water-gellable polysaccharides. For example, hyaluronic acid, chitosan and/or a fucose-rich polysaccharide, which is available under the name Fucogel™ 1000 from SOLABIA S.A., are especially advantageous.

### Actives

Within the context of the present invention, water-soluble antioxidants can be used particularly advantageously, such as, for example, vitamins, e.g. ascorbic acid and derivatives thereof. Vitamin E and derivatives thereof, and also vitamin A and derivatives thereof are very particularly advantageous.

Further advantageous active ingredients in the composition according to the invention are α-hydroxy acid, such as glycolic acid, lactic acid, malic acid, tartaric acid, citric acid and mandelic acid, β-hydroxy acid, such as salicylic acid, and acylated derivatives thereof, 2-hydroxyalkanoic acid and its derivatives; peptides, enzymes, natural active ingredients and/or derivatives thereof, such as, for example, alpha-lipoic acid, folic acid, phytoene, D-biotin, coenzyme Q10, alpha-glucosylrutin, carnitine, carnosine, natural and/or synthetic isoflavonoids, creatin, creatinine, taurine and/or [beta]-alanine and also 8-hexadecene-1,16-dicarboxylic acid (dioic acid, CAS number 20701-68-2; provisional INCI name Octadecenedioic acid) and/or Licochalcon A and the plant extracts.

### Film Formers

If appropriate, the cosmetic compositions according to the invention comprise further film formers, addiotinally to the polyurethane film former from the invention. The film former or the film formers are advantageously selected according to the invention from the group of water-soluble or water-dispersible polyurethanes, polyacrylates, the polyureas, silicone resins and/ or polyesters, and also the nonionic, anionic, amphoteric and/or cationic polymers and their mixtures.

Advantageous nonionic polymers which may be present in the compositions according to the invention alone or in a mixture, preferably also with anionic and/or amphoteric and/or zwitterionic polymers, are selected from:
polyalkyloxazolines;
vinyl acetate homopolymers or copolymers. These include, for example, copolymers of vinyl acetate and acrylic acid esters, copolymers of vinyl acetate and ethylene, copolymers of vinyl acetate and maleic acid esters;
acrylic acid ester copolymers, such as, for example, the copolymers of alkyl acrylate and alkyl methacrylate, copolymers of alk)'l acrylate and urethanes;
copolymers of acrylonitrile and nonionic monomer selected from butadiene and (meth)acrylate;
styrene homopolymers and copolymers. These include, for example, homopolystyrene, copolymers of styrene and alkyl (meth)acrylate, copolymers of styrene, alkyl methacrylate and alkyl acrylate, copolymers of styrene and butadiene, copolymers of styrene, butadiene and vinylpyridine:
   polyamides;
   vinyllactam homopolymers or copolymers, such as vinylpyrrolidone homopolymers or copolymers; these include, for example, polyvinylpyrrolidone, polyvinylcaprolactam, copolymers of N-vinylpyrrolidone and vinyl acetate and/or vinyl propionate in various concentration ratios, polyvinylcaprolactam, polyvinylamides and salts thereof, and copolymers of vinylpyrrolidone and dimethylaminoethyl methacrylate, terpolymers of vinylcaprolactam, vinylpyrrolidone and dimethylaminoethylmethacrylate;
   polysiloxanes;
   homopolymers of N-vinylformamide

Particularly preferred nonionic polymers are acrylic acid ester copolymers, homopolymers and copolymers of vinylpyrrolidone and polyvinylcaprolactam. Very particularly preferred nonionic polymers are homopolymers of vinylpyrrolidone, e.g. Luviskol® K from BASF, copolymers of vinylpyrrolidone and vinyl acetate, e.g. Luviskol® VA grades from BASF, terpolymers of vinylpyrrolidone, vinyl acetate and propionate, such as, for example, Luviskol® VAP from BASF and polyvinylcaprolactalam

Advantageous anionic polymers are homopolymers or copolymers with monomer units containing acid groups which are optionally copolymerized with comonomers which contain no acid groups. Suitable monomers are unsaturated, free-radically polymerizable compounds which have at least one acid group, in particular carboxylic acid, sulphonic acid or phosphonic acid.

Advantageous anionic polymers comprising carboxylic acid groups are:
acrylic acidormethacrylic acidhomopolymers or copolymers or the salts thereof. These include, for example, the copolymers of acrylic acid and acrylamides and/or sodium salts thereof, copolymers of acrylic acid and/or methacrylic acid and an unsaturated monomer selected from ethylene, styrene, vinyl ester, acrylic acid ester, methacrylic acid ester, optionally ethoxylated compounds, copolymers of vinylpyrrolidones, acrylic acid and C1-C20 alky'l methacrylates, e.g. Acrylidone® LM from ISP, copolymers of methacrylic acid, ethyl acrylates and tert-butyl acrylates, e.g. Luvimer® 100 P from BASF;
crotonic acid derivative homopolymers or copolymers or the salts thereof. These include, for example, vinyl acetate/crotonic acid, vinyl acetate/acrylate and/or vinyl acetate/vinyl neodecanoate/crotonic acid copolymers and sodium acrylate/vinyl alcohol copolymers;
unsaturated C4-C8 carboxylic acid derivatives or carboxylic anhydride copolymers selected from copolymers of maleic acid or maleic anhydride or fumaric acid or fumaric anhydride or itaconic acid or itaconic anhydride and at least one monomer selected from vinyl esters, vinyl ethers, vinyl-halogen derivatives, phenylvinyl derivatives, acrylic acid, acrylic acid esters or copolymers of maleic acid or maleic anhydride or fumaric acid or fumaric anhydride or itaconic acid or itaconic anhydride and at least one monomer selected from allyl esters, methallyl esters and optionally acrylamides, methacrylamides, alpha-olefin, acrylic acid esters, methacrylic acid esters, vinylpyrrolidones. Other preferred polymers are methyl vinyl ether/maleic acid copolymers which are formed by hydrolysis of vinyl ether/maleic anhydride copolymers. These polymers may also be partially esterified (ethyl, isopropyl or butyl esters) or partially amidated.
water-soluble or -dispersible anionic polyurethanes, e.g. Luviset® PUR trom BASF, which could beor not different from the polyurethanes according to the invention,
where this list is of course not intended to be limiting.

Advantageous anionic polymers comprising sulphonic acid group are salts of polyvinylsulphonic acid, salts of polystyrenesulphonic acid, such as, for example, sodium polystyrene sulphonate or salts of polyacrylamidosulphonic acid. Particularly advantageous anionic polymers are acrylic acid copolymers, crotonic acid derivative copolymers, copolymers of maleic acid and maleic anhydride or nunaric acid and fumaric anhydride or itaconic acid and itaconic anhydride and at least one monomer selected trom vinyl esters, vinyl ethers, vinyl halogen derivatives, phenylvinyl derivatives, acrylic acid, acrylic acid esters and salts of polystyrene sulphonic acid. Very particularly advantageous anionic polymers are acrylate copolymers, e.g. Luvimer from BASF, ethyl acrylate/N-tert-butylacrylamide/acrylic acid copolymers ULTRAHOLD® STRONG from BASF, VA/crotonate/vinyl neodecanoate copolymer, e.g. Resyn 28-2930 from AkzoNobel, copolymers such as, for example, copolymers of methyl vinyl ether and maleic anhydride partially esterified e.g. GANTREZ® from ISP and sodium polystyrene sulphonates, e.g. Flexan 130 from AkzoNobel.

Advantageous amphoteric polymers can be selected from the polymers which contain units A and B distributed randomly in the polymer chain, where A is a unit which is derived from a monomer with at least one basic nitrogen atom, and B is a unit which originates from an acidic monomer which has one or more carboxy groups or sulphonic acid groups, or A and B may be groups which are derived from zwitterionic carboxybetaine monomers or sulphobetaine monomers; A and B can also be a cationic polymer chain which contains primary, secondary, tertiary or quaternary groups, in which at least one amino group carries a carboxy group or sulphonic acid group which is bonded via a hydrocarbon group, or Band C are part of a polymer chain with ethylene-a,B-dicarboxylic acid unit in which the carboxylic acid groups have been reacted with a polyamine which contains one or more primary or secondary amino groups.

Particularly advantageous amphoteric polymers are:
polymers which are formed during the copolymerization of a monomer derived trom a vinyl compound with carboxy group, such as, in particular, acrylic acid, methacrylic acid, maleic acid, a-chloroacrylic acid, and a basic monomer which is derived from a vinyl compound which is substituted and comprises at least one basic atom, such as, in particular, dialkylaminoalkyl methacrylate and acrylate, dialkylaminoalkylmethacrylamide and -acrylamide. Such compounds have been described in the U.S. Pat. No. 3,836,537.
polymers with units which are derived from: a) at least one monomer which is selected from the acrylamides or methacrylamides which are substituted on the nitrogen atom with an alkyl group, b) at least one acidic comonomer which contains one or more reactive carboxy groups, and c) at least one basic comonomer, such as esters of acrylic acid and methacrylic acid with primary, secondary, tertiary and quaternary amino substituents and the quaternization product of dimethylaminoethyl methacrylate with dimethyl sulphate or diethyl sulphate.

N-substituted acrylamides or methacrylamides particularly preferred according to the invention are compounds whose alkyl groups contain 2 to 12 carbon atoms, particularly N -ethylacrylamide, N-t-butylacrylamide, N-t-octylacrylamide, N-octylacrylamide, N-decylacrylamide, N-dodecylacrylamide and the corresponding methacrylamides.

The acidic comonomers are selected in particular from acrylic acid, methacrylic acid, crotonic acid, itaconic acid, maleic acid, fumaric acid and the alkyl monoesters having 1 to 4 carbon atoms of maleic acid, maleic anhydride, fumaric acid or fumaric anhydride.

Preferred basic comonomers are aminoethyl methacrylate, butylaminoethyl methacrylate, N,N-dimethylaminoethyl methacrylate, N -t -butylaminoethylmethacrylate.

Crosslinked and completely or partially acylated polyaminoamides which are derived from polyaminoamides of the following general fornmla:

-[CO-R∼O-Z1

in which R is a divalent group which is derived from a saturated dicarboxylic acid, an aliphatic mono-or dicarboxylic acid with ethylenic double bond, an ester of these acids with a lower alkanol having 1 to 6 carbon atoms or a group which is formed during the addition of one of these acids onto a bis primary or bis secondary amine, and Z is a group which is derived from a bisprimary, mono-or bis-secondary polyalkylenepolyamine, and preferably: a) in quantitative fractions of from 60 to 100 mol % the groups -NH-[(CH₂)ₓ-NH-]ₚ- where x=2 and p=2 or 3 or x=3 and p=2, where this group is derived from diethylenetriamine, triethylenetetramine or dipropylenetriamine; b) in quantitative fractions of from 0 to 40 mol % the group -NH-[(CH₂)ₓ-NH-]ₚ- in which x=2 and p=1, which is derived from ethylenediamine, or the group which originates from piperazine: 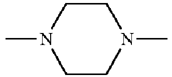 c) in quantitative fractions of from 0 to 20 mol %, the group -H-(CH₂)₆-NH-, which is derived from hexamethylenediamine, where these polyaminoamides are crosslinked by addition of a bifunctional crosslinking agent which is selected from the epihalohydrins, diepoxides, dianhydrides and bis-unsaturated derivatives, in an amount of from 0.025 to 0.35 mol of crosslinking agent per amino group of the polyaminoamide and are acylated with acrylic acid, chloroacetic acid or an alkanesulphone or salts thereof.

The saturated carboxylic acids are preferably selected from the acids having 6 to 10 carbon atoms, such as adipic acid, 2,2,4-trimethyladipic acid and 2,4, 4,-trimethyladipic acid. terephthalic acid; acids with ethylenic double bond, such as, for example, acrylic acid, methacrylic acid and itaconic acid.

The alkanesultones used in the acylation are preferably propanesultone or butanesultone, the salts of the acylating agents are preferably the sodium salts or potassium salts.

Polymers with zwitterionic units of the following formula: 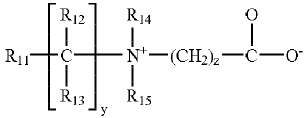 in which R₁₁ is a polymerizable unsaturated group, such as acrylate, methacrylate, acrylamide or methacrylamide, y and z are integers from1 to 3, R₁₂ and R₁₃ are a hydrogen atom, methyl, ethyl or propyl R₁₄ and R₁₅ are a hydrogen atom or an alkyl group which is selected such that the sum of the carbon atoms R₁₄ and R₁₅ does not exceed 10.

Polymers which contain such units can also have units which originate from non-zwitterionic monomers, such as dimethyl-and diethylaminoethyl acrylate or dimethyl-and diethylaminoethylmethacrylate or alkyl acrylates or alkylmethacrylates, acrylamides or methacrylamides or vinyl acetate.

Polymers which are derived from chitosan and contain monomer units which correspond to the following formulae: 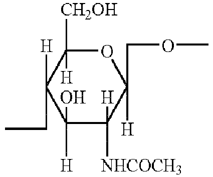 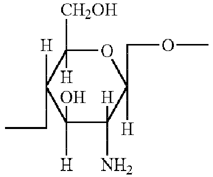 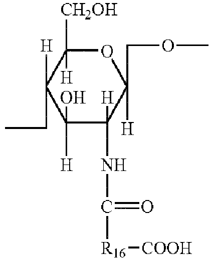 where the first unit is present in quantitative fractions of from 0 to 30%, the second unit is present in quantitative fractions of from 5 to 50% and the third unit is present in quantitative fractions of from 30 to 90%, with the proviso that in the third unit R₁₆ is a group of the following formula: 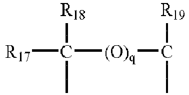 in which: if q=O, the groups R₁₇, R₁₈ and R₁₉, which are identical or different, are in each case a hydrogen atom, methyl, hydroxy, acetoxy or amino, a monoalkylamine radical or a dialkylamine radical which optionally interrupted by one or more nitrogen atoms and/or optionally by one or more groups, amino, hydroxy, carboxy, alkylthio, sulphonic acid, alkylthio, the alkyl group of which carries an amino radical, where at least one of the groups R₁₇, R₁₈ and R₁₉ is in this case a hydrogen atom; or if q=1, the groups R₁₇, R₁₈ and R₁₉ are in each case a hydrogen atom, and the salts which form these compounds with bases or acids.

Polymers which correspond to the following general formula and which are described, for example, in the French patent 1 400366: 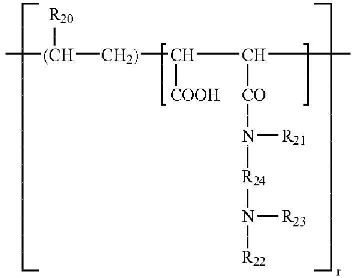 in which R₂₀ is a hydrogen atom, CH₃O, CH₃CH₂O or phenyl, R21 is a hydrogen atom or a lower alkyl group, such as methyl or ethyl, R₂₂ is a hydrogen atom or a lower C₁₋₆-alkyl group, such as methyl or ethyl, R₂₃ is a lower C₁₋₆-alkyl group, such as methyl or ethyl or a group of the formula: - R₂₄-N(R₂₂)₂, where R₂₄ is a group -CH₂-CH₂, --CH₂--CH₂--CH₂-or --CH₂--CH(CH₃)-and where R₂₂ has the meanings given above.

Polymers which can be formed during the N-carboxyalkylation of chitosan, such as N-carboxymethylchitosan or N-carboxybutylchitosan.

Amphoteric polymers of the type -D-X-D-X, which are selected from: a) polymers which are formed by the action of chloroacetic acid or sodium chloroacetate on compounds with at least one unit -D-X-D-X:
in which D is the group

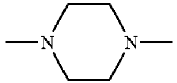

And X is the symbols E or E', where E or E', which are identical or different, are a divalent group which is a straight-chain or branched alkylene group having up to 7 carbon atoms in the main chain which is present in unsubstituted form or is substituted by hydroxy groups and can contain one or more oxygen atoms, nitrogen atoms or sulphur atoms and 1 to 3 aromatic and/or heterocyclic rings; where the oxygen atoms, nitrogen atoms and sulphur atoms are present in the form of the following groups: ether, thioether, sulphoxide, sulphone, sulphonium, alkylamine, alkenylamine, hydroxy, benzylamine, anline oxide, quaternary annnonium, amide, imide, alcohol, ester and/or urethane.

### b) polymers of the fonnula -D-X-D-X, in which D is the group

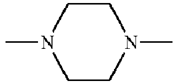

and X is the symbol E or E' and at least once E'; where E has the meanings given above and E' is a divalent group which is a straight-chain or branched alkylene group having up to 7 carbon atoms in the main chain, which is present in unsubstituted fonn or is substituted by one or more hydroxy groups and contains one or more nitrogen atoms, where the nitrogen atom is substituted by an alkyl group which is optionally interrupted by an oxygen atom and obligatorily comprises one or more carboxy functions or one or more hydroxy functions and is betainized through reaction with chloroacetic acid or sodium chloroacetate.

Alkyl(C₁₋₅) vinyl ether/maleic anhydride copolymers which in part are partially modified by semi-amidation with an N,N-dialkylaminoalkylamine, such as N,N-dimethylaminopropylamine or an N,N-dialkylaminoalcohol. These polymers can also contain further comonomers, such as vinylcaprolactam.

Very particularly advantageous amphoteric polymers are, for example, the copolymers octylacrylamide/acrylates/butylaminoethyl methacrylate copolymers which are connnercially available under the names AMPHOMER®, AMPHOMER® LV 71 or BALANCE® 47 fromAkzoNobel, and methyl methacrylate/methyl dimethylcarboxymethylanmonium ethyl methacrylate copolymers.

### Conditioning Agents

If appropriate, the cosmetic compositions according to the invention comprise a conditioning agent. Conditioning agents preferred according to the invention are, for example, all compounds which are listed in the International Cosmetic Ingredient Dictionary and Handbook (Volume 4, editor: R. C. Pepe, 1. A. Wenninger, G. N. McEwen, The Cosmetic, Toiletry, and Fragrance Association, 9th edition, 2002) under Section 4 under the keywords Hair Conditioning Agents, Humectants, Skin-Conditioning Agents, SkinConditioning Agents-Emollient, Skin-Conditioning Agents-Humectant, SkinConditioning Agents-Miscellaneous, Skin-Conditioning Agents-Occlusive and Skin Protectants, and also all of the compounds listed in EP-A 934 956 (pp. 11-13) under "water soluble conditioning agent" and "oil soluble conditioning agent".

Particularly advantageous conditioning substances are, for example, the compounds referred to in accordanee with INCI as Polyquaternium (in particular polyquaternium-1 to polyquaternium-103).

Suitable conditioning agents include, for example, also polymeric quaternary ammonium compounds, cationic cellulose derivatives, chitosan derivatives, guar gum derivatives and polysaccharides, in particular guar hydroxypropylammonium chloride (e.g. Jaguar® from Rhodia).

Further conditioning agents advantageous according to the invention are non-ionic poly-N-vinylpyrrolidone/ polyvinyl acetate copolymers (e.g. Luviskol® VA 64 from BASF AG), anionic acrylate copolymers (e.g. Luvifiex®Soft from BASF AG), and/or amphoterie amide/acrylate/methacrylate copolymers Ce.g. Amphomer® from National Starch). Further conditioning agents are quaternized silicones.

The present invention is illustrated by reference to examples, although these are not to be understood as being limiting. Unless stated otherwise, all of the quantitative data, fractions and percentages are based on the weight and the total amount or on the total weight of the compositions.

### Examples:

Unless noted otherwise, all of the analytical measurements refer to measurements at temperatures of 23°C.

The solid or solid-body contents are determined by heating a weighed sample at 125°C to constant weight. At constant weight, the solid-body content is calculated by reweighing the sample.

Unless expressly mentioned otherwise, NCO contents were determined volumetrically in accordance with DIN-EN ISO 11909.

The control on free NCO groups was carried out by means of IR spectroscopy (band at 2260 cm⁻¹).

These stated viscosities were determined by means of rotary viscometry in accordance with DIN 53019 at 23°C using a rotary viscometer from Anton Paar Germany GmbH, Ostfildem, Germany.

The average particle sizes (the number-average is given) of the polyurethane dispersions were determined following dilution with deionized water by means of laser correlation spectroscopy (instrument: Malvern Zetasizer 1000, Malver Inst. Limited).

### Substances used and abbreviations:

| | |
|---|---|
| Diaminosulphonate: | NH₂-CH₂CH₂-NH-CH₂CH₂-SO₃Na (45% strength in water) |
| Desmophen® 2020/C2200: | Polycarbonate polyol, OH number 56 mg of KOH/g, number-average molecular weight 2000 g/mol (Bayer MaterialScience AG, Leverkusen, Germany) |
| PolyTHF® 2000: | Polytetramethylene glycol polyol, OH number 56 mg of KOH/g, number-average molecular weight 2000 g/mol (BASF AG, Ludwigshafen, Germany) |
| PolyTHF® 1000: | Polytetramethylene glycol polyol, OH number 112 mg of KOH/g, number-average molecular weight 1000 g/mol (BASF AG, Ludwigshafen, Germany) |
| Polyether LB 25: | monofunctional polyether based on ethylene oxide/propylene oxide of number-average molecular weight 2250 g/mol, OH number 25 mg of KOH/g (Bayer MaterialScience AG, Leverkusen, Germany) |

### Example 1: Polyurethane dispersion 1

987.0 g of PolyTHF® 2000 (component A2)), 375.4 g of PolyTHF® 1000 (component A2)), 761.3 g of Desmophen® C2200 (component A2)) and 44.3 g of polyether LB 25 (component A4)) were heated to 70°C in a standard stirring apparatus. Then, a mixture of 237.0 g of hexamethylene diisocyanate (component A1)) and 313.2 g of isophorone diisocyanate (component A1)) was added and the mixture was stirred at 120°C until the theoretical NCO value was reached. The finished prepolymer was dissolved with 4830 g of acetone and in so doing cooled to 50°C, and then a solution of 25.1 g of ethylenediamine (component B1)), 116.5 g of isophoronediamine (component B1)), 61.7 g of diaminosulphonate (component B2)) and 1030 g of water was metered in. The after stirring time was 10 min. The mixture was then dispersed by adding 1250 g of water. The solvent was removed by distillation in vacuo.

The resulting white dispersion had the following properties:

| | |
|---|---|
| Solids content: | 61 % |
| Particle size (LCS): | 312 nm |
| Viscosity (viscometer, 23°C): | 241 mPas |
| pH (23°C): | 6.02 |

### Example 2: Polyurethane dispersion 2

450 g of PolyTHF® 1000 (component A2)) and 2100 g of PolyTHF® 2000 (component A2)) were heated to 70°C. Then, a mixture of 225.8 g of hexamethylene diisocyanate (component A1)) and 298.4 g of isophorone diisocyanate (component A1)) was added and the mixture was stirred at 100-115°C until the actual NCO value had dropped below the theoretical NCO value. The finished prepolymer was dissolved with 5460 g of acetone at 50°C and then a solution of 29.5 g of ethylenediamine (component B1)), 143.2 g of diaminosulphonate (component B2)) and 610 g of water was metered in. The after stirring time was 15 min. The mixture was then dispersed by adding 1880 g of water. The solvent was removed by distillation in vacuo and a storage-stable dispersion was obtained.

| | |
|---|---|
| Solids content: | 56% |
| Particle size (LCS): | 276 nm |
| Viscosity: | 1000 mPas |

### Example 3: Polyurethane dispersion 3

1649.0 g of a polyester of adipic acid, hexanediol and neopentyl glycol with an average molecular weight of 1700 g/mol (component A2)) were heated to 65°C. Then, 291.7 g of hexamethylene diisocyanate (component A1)) were added and the mixture was stirred at 100-115°C until the actual NCO value had dropped below the theoretical NCO value. The finished prepolymer was dissolved with 3450 g of acetone at 50°C and then a solution of 16.8 g of ethylenediamine (component B1)), 109.7 g of diaminosulphonate (component B2)) and 425 g of water was metered in. The after stirring time was 15 min. The mixture was then dispersed by adding 1880 g of water. The solvent was removed by distillation in vacuo and a storage-stable dispersion was obtained.

| | |
|---|---|
| Solids content: | 42% |
| Particle size (LCS): | 168 nm |
| Viscosity: | 425 mPas |
| pH: | 7.07 |

### Example 4: Polyurethane dispersion 4

340 g of a polyester of adipic acid, hexanediol and neopentyl glycol with an average molecular weight of 1700 g/mol (component A2)) were heated to 65°C. Then, 60.1 g of hexamethylene diisocyanate (component A1)) were added and the mixture was stirred at 105°C until the actual NCO value had dropped below the theoretical NCO value. The finished prepolymer was dissolved with 711 g of acetone at 50°C and then a solution of 2.1 g of ethylenediamine (component B1)), 32.4 g of diaminosulphonate (component B2)) and 104.3 g of water was metered in. The after stirring time was 15 min. The mixture was then dispersed by adding 1880 g of water. The solvent was removed by distillation in vacuo and a storage-stable dispersion was obtained.

| | |
|---|---|
| Solids content: | 40% |
| Particle size (LCS): | 198 nm |
| Viscosity: | 700 mPas |
| pH: | 6.31 |

### Example 5: Polyurethane dispersion 5

450 g of PolyTHF® 1000 (component A2)) and 2100 g of PolyTHF® 2000 (component A2)) were heated to 70°C. Then, a mixture of 225.8 g of hexamethylene diisocyanate (component A1)) and 298.4 g of isophorone diisocyanate (component A1)) was added and the mixture was stirred at 100-115°C until the actual NCO value had dropped below the theoretical NCO value. The finished prepolymer was dissolved with 5460 g of acetone at 50°C and then a solution of 351 g of diaminosulphonate (component B2)) and 610 g of water was metered in. The after stirring time was 15 min. The mixture was then dispersed by adding 1880 g of water. The solvent was removed by distillation in vacuo and a storage-stable dispersion was obtained.

| | |
|---|---|
| Solids content: | 40% |
| Viscosity: | 1370 mPas |

### APPLICATION EXAMPLES:

The quantitative data in the tables below are % by weight based on the total amount of the compositions.

| **Formulation** | | **1** | **2** |
|---|---|---|---|
| **Phase** | **INCI Designation** | **%(w/w)** | **%(w/w)** |
| A | Deionized Water | Add to 100.00 | Add to 100.00 |
| A | Disodium EDTA | 0.10 | 0.10 |
| A | Allantoin | 0.25 | 0.25 |
| A | Glycerin | 3.00 | 3.00 |
| A | Diazolidinyl Urea (and) Iodopropynyl Butylcarbamate | 0.25 | 0.25 |
| A | Ammonium Acryloyldimethyltaurate/VP Copolymer | 1.00 | 1.00 |
| B | Cyclopentasiloxane (and) Cyclohexasiloxane | 5.00 | 5.00 |
| B | Isododecane | 5.00 | 5.00 |
| B | Caprylic/Capric Triglyceride | 5.00 | 5.00 |
| B | Isododecane (and) Polysilicone-11 | 8.00 | 8.00 |
| B | Polysorbate 20 | 1.00 | 1.00 |
| B | Polyacrylamide (and) C13-14 Isoparaffin (and) Laureth-7 | 3.00 | 3.00 |
| C | Salicylic Acid | 2.00 | 2.00 |
| C | Butylene Glycol | 5.00 | 5.00 |
| C | Dimethyl Isosorbide | 5.00 | 5.00 |
| C | Bisabolol | 0.50 | 0.50 |
| C | PPG-5-Ceteth-20 | 3.00 | 3.00 |
| C | Ammonium Hydroxide | 0.50 | 0.50 |
| D | Water and Butylene Glycol and Glycerin (and)Avena Sativa (Oat) Kernal Extract | 2.00 | 2.00 |
| D | Glycerin and Water and Camellia Oleifera Leaf Extract | 0.50 | 0.50 |
| E | Polyurethane dispersion according to the invention (based on solid % by weight) | | 2.00 |
| | | **100.00** | **100.00** |

### Procedure:

- Add Phase A ingredients in main vessel at RT sequentially, as listed. Mix with rapid agitation until thick and uniform.
- In separate vessel combine Phase B ingredients. Mix until uniform. Add to Phase A.
- Prepare Phase C by combining all ingredients except for Ammonium Hydroxide. Mix until uniform and clear.Phase may be warmed to 35°C to facilitate dissolution. Add Ammonium Hydroxide. Add to main vessel.
- Adjust the pH to 4,0 - 4,5

### Study design:

The goal was to investigate the role of the polyurethane dispersion according to the invention in the treatment of acne. The influence of polyurethane dispersion according to the invention on efficacy of skincare products (formulation 2) was in vivo determined and compared to skincare product without a polyurethane dispersion (Formulation 1)
- 7 subjects (6 male and 1 female) with moderate-mild acne were selected for the test panel.
- The infrascapular area of the back to the right or left side of the midline was used as a test area. The acne lesion was centered within a - 2 cm by 2 cm test site. Test sites were far enough apart that the products could not interact due to migration.
- Test products were applied once daily for four consecutive days via plastic volumetric syringe at a concentration of 2 mg/cm².
- The size and type of acne lesions was recorded and graded.

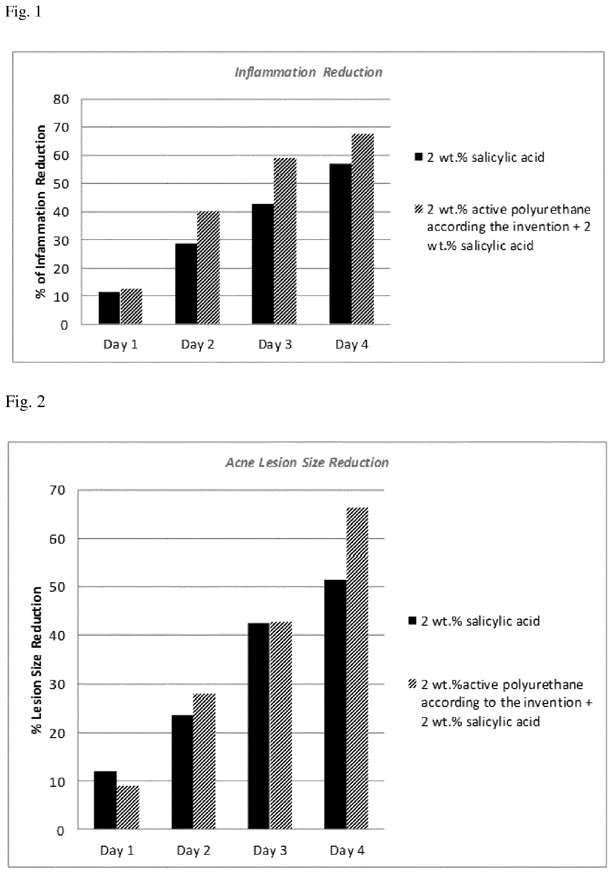

The polyurethane dispersion according to the invention improves efficacy of the ant acne skincare product by significantly reducing lesion size and inflammation (Figures 1 and 2).

## Claims

1. An aqueous composition for the treatment of acne comprising at least one aqueous polyurethane dispersion and salicylic acid and/or at least one salicylic acid derivative, **characterized in that** the salicylic acid derivative is selected from the group consisting of acetaminosalol, aspirin, balsalazide, benorylate, calcium acetylsalicylate, diflunisal, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salicylamide O-acetic acid, salicylsulfuric acid, salsalate, sodium salicylate and sulfasalazine and the salicylic acid and/or at least one salicylic acid derivative is present in an amount of 0.1 to 10 % by weight, related to the total weight of the composition and wherein aqueous polyurethane dispersion comprises an anionically hydrophilized polyurethane polymer, which is obtainable by reacting one or more isocyanate-functional polyurethane prepolymers A) which have neither ionic nor ionogenic groups, with one or more amino-functional compounds B), which include at least one amino-functional compound B2) which has ionic and/or ionogenic groups.

2. The aqueous composition according to claim 1, **characterized in that** the anionically hydrophilized polyurethane is present in an amount of 0.1 to 15 % by weight, related to the total weight of the composition.

3. The aqueous composition according to one of claims 1 or 2, **characterized in that** the amino-functional compounds B) are selected from primary and/or secondary amines and/or diamines.

4. The aqueous composition according to claim 2, **characterized in that** the amino-functional compounds B) include at least one diamine.

5. The aqueous composition according to one of claims 1 to 4, **characterized in that** the ionic and/or ionogenic group used is a sulphonate or the sulphonic acid group.

6. The aqueous composition according to one of claims 1 to 5, **characterized in that** the amino-functional compounds B) include both amino-functional compounds B2) which have ionic and/or ionogenic groups, and also amino-functional compounds B1) which have no ionic or ionogenic group.

7. The aqueous composition according to one of claims 1 to 6, **characterized in that** the prepolymers A) used for the preparation of the polyurethanes are obtainable by reacting one or more polyols selected from the group which consists of polyether polyols, polycarbonate polyols, polyether polycarbonate polyols and/or polyester polyols with polyisocyanates.

8. A skin care product comprising at least one composition according to one or more of claims 1 to 7.

## Patentansprüche

1. Wässrige Zusammensetzung zur Behandlung von Akne, enthaltend mindestens eine wässrige Polyurethandispersion und Salicylsäure und/oder mindestens ein Salicylsäurederivat, **dadurch gekennzeichnet, dass** das Salicylsäurederivat aus der Gruppe bestehend aus Acetaminosalol, Aspirin, Balsalazid, Benorylat, Calciumacetylsalicylat, Diflunisal, Fendosal, Gentisinsäure, Glykolsalicylat, Imidazolsalicylat, Lysinacetylsalicylat, Mesalamin, Morpholinsalicylat, 1-Naphthylsalicylat, Olsalazin, Parsalmid, Phenylacetylsalicylat, Phenylsalicylat, Salicylamid-O-essigsäure, Salicylschwefelsäure, Salsalat, Natriumsalicylat und Sulfasalazin ausgewählt ist und die Salicylsäure und/oder mindestens ein Salicylsäurederivat in einer Menge von 0,1 bis 10 Gew.-%, bezogen auf das Gesamtgewicht der Zusammensetzung, vorliegt und wobei die wässrige Polyurethandispersion ein anionisch hydrophiliertes Polyurethanpolymer enthält, das erhältlich ist durch Umsetzung eines oder mehrerer isocyanatfunktioneller Polyurethanprepolymere A), die weder ionische noch ionogene Gruppen aufweisen, mit einer oder mehreren aminofunktionellen Verbindungen B), die mindestens eine aminofunktionelle Verbindung B2), die ionische und/oder ionogene Gruppen aufweist, umfassen.

2. Wässrige Zusammensetzung nach Anspruch 1, **dadurch gekennzeichnet, dass** das anionisch hydrophilierte Polyurethan in einer Menge von 0,1 bis 15 Gew.-%, bezogen auf das Gesamtgewicht der Zusammensetzung, vorliegt.

3. Wässrige Zusammensetzung nach einem der Ansprüche 1 oder 2, **dadurch gekennzeichnet, dass** die aminofunktionellen Verbindungen B) aus primären und/oder sekundären Aminen und/oder Diaminen ausgewählt sind.

4. Wässrige Zusammensetzung nach Anspruch 2, **dadurch gekennzeichnet, dass** die aminofunktionellen Verbindungen B) mindestens ein Diamin umfassen.

5. Wässrige Zusammensetzung nach einem der Ansprüche 1 bis 4, **dadurch gekennzeichnet, dass** es sich bei der verwendeten ionischen und/oder ionogenen Gruppe um eine Sulfonat- bzw. die Sulfonsäuregruppe handelt.

6. Wässrige Zusammensetzung nach einem der Ansprüche 1 bis 5, **dadurch gekennzeichnet, dass** die aminofunktionellen Verbindungen B) sowohl aminofunktionelle Verbindungen B2), die ionische und/oder ionogene Gruppen aufweisen, als auch aminofunktionelle Verbindungen B1), die keine ionische oder ionogene Gruppe aufweisen, umfassen.

7. Wässrige Zusammensetzung nach einem der Ansprüche 1 bis 6, **dadurch gekennzeichnet, dass** die für die Herstellung der Polyurethane verwendeten Prepolymere A) durch Umsetzung eines oder mehrerer Polyole aus der Gruppe bestehend aus PolyetherPolyolen, Polycarbonat-Polyolen, Polyether-Polycarbonat-Polyolen und/oder Polyester-Polyolen mit Polyisocyanaten erhältlich sind.

8. Hautpflegeprodukt, enthaltend mindestens eine Zusammensetzung nach einem oder mehreren der Ansprüche 1 bis 7.

## Revendications

1. Composition aqueuse destinée au traitement de l'acné, comprenant au moins une dispersion aqueuse de polyuréthane et de l'acide salicylique et/ou au moins un dérivé d'acide salicylique, **caractérisée en ce que** le dérivé d'acide salicylique est choisi dans le groupe constitué par l'acétaminosalol, l'aspirine, le balsalazide, le bénorilate, l'acétylsalicylate de calcium, le diflunisal, le fendosal, l'acide gentisique, le salicylate de glycol, le salicylate d'imidazole, l'acétylsalicylate de lysine, la mésalamine, le salicylate de morpholine, le salicylate de 1-naphtyle, l'olsalazine, le parsalmide, l'acétylsalicylate de phényle, le salicylate de phényle, l'acide O-acétique salicylamide, l'acide salicylsulfurique, le salsalate, le salicylate de sodium et la sulfasalazine, et l'acide salicylique et/ou au moins un dérivé d'acide salicylique est présent selon une quantité allant de 0,1 à 10% en poids, par rapport au poids total de la composition et où la dispersion aqueuse de polyuréthane comprend un polymère de polyuréthane hydrophilisé de manière anionique, qui peut être obtenu par la réaction d'un ou plusieurs prépolymères de polyuréthane à fonction isocyanate A) qui n'ont ni groupement ionique, ni groupement ionogène, avec un ou plusieurs composés à fonction amino B), qui comportent au moins un composé à fonction amino B2) qui possède des groupements ioniques et/ou ionogènes.

2. Composition aqueuse selon la revendication 1, **caractérisée en ce que** le polyuréthane hydrophilisé de manière anionique est présent selon une quantité allant de 0,1 à 15% en poids, sur la base du poids total de la composition.

3. Composition aqueuse selon l'une des revendications 1 ou 2, **caractérisée en ce que** les composés à fonction amino B) sont choisis parmi les amines et/ou les diamines primaires et/ou secondaires.

4. Composition aqueuse selon la revendication 2, **caractérisée en ce que** les composés à fonction amino B) comportent au moins une diamine.

5. Composition aqueuse selon l'une des revendications 1 à 4, **caractérisée en ce que** le groupement ionique et/ou ionogène utilisé est un sulfonate ou un groupement acide sulfonique.

6. Composition aqueuse selon l'une des revendications 1 à 5, **caractérisée en ce que** les composés à fonction amino B) comportent les composés à fonction amino B2) qui possèdent des groupements ioniques et/ou ionogènes, ainsi que les composés à fonction amino B1) qui ne possèdent aucun groupement ionique ou ionogène.

7. Composition aqueuse selon l'une des revendications 1 à 6, **caractérisée en ce que** les prépolymères A) utilisés pour la préparation des polyuréthanes peuvent être obtenus par la réaction d'un ou plusieurs polyols choisis dans le groupe constitué par les polyéther-polyols, les polycarbonate-polyols, les polyéther-polycarbonate-polyols et/ou les polyester-polyols avec des polyisocyanates.

8. Produit de soin de la peau, comprenant au moins une composition selon l'une ou plusieurs parmi les revendications 1 à 7.
